# EUROPEAN PATENT SPECIFICATION

(11) **EP 2 535 052 B1**
(45) Date of publication and mention of the grant of the patent: **07.09.2016**
(21) Application number: 11739852.9
(22) Date of filing: 04.02.2011
(51) Int. Cl.: A61K 35/14, A61K 35/28, A61K 35/44, A61P 35/00, A61P 37/04, C12N 5/0783, A61K 35/12

(54) **METHOD FOR PRODUCING NK CELL ENHANCEMENT-TYPE BLOOD PRODUCT**
VERFAHREN ZUR HERSTELLUNG EINES DURCH NK-ZELLEN ERWEITERTEN BLUTPRODUKTS
PROCÉDÉ DE PRODUCTION D'UN PRODUIT SANGUIN DU TYPE À AMPLIFICATION DES CELLULES NK

(30) Priority: 08.02.2010 JP 2010025940
(43) Date of publication of application: 19.12.2012
(73) Proprietor: Biotherapy Institute Of Japan, Tokyo 135-0051 (JP)
(72) Inventor: DENG Xuewen, Chengdu 610021 (CN); TERUNUMA Hiroshi, Tokyo 113-0033 (JP); NIEDA Mie, Tokyo 150-0013 (JP)
(74) Representative: Herzog, Fiesser & Partner Patentanwälte PartG mbB
(86) International application number: PCT/JP2011/052314
(87) International publication number: WO 2011/096504

(56) References cited:
- WO-A1-2006/006720
- WO-A1-2009/151183
- JP-A- 2007 297 291
- JP-A- 2008 508 244
- JP-B1- 4 281 071
- PORTEVIN DAMIEN ET AL: "Regulatory activity of azabisphosphonate-capped dendrimers on human CD4+ T cell proliferation enhances ex-vivo expansion of NK cells from PBMCs for immunotherapy", JOURNAL OF TRANSLATIONAL MEDICINE, BIOMED CENTRAL, LONDON, GB, vol. 7, no. 1, 24 September 2009 (2009-09-24), page 82, XP021063598, ISSN: 1479-5876, DOI: 10.1186/1479-5876-7-82
- ZHAO-LIANG HU ET AL: "Effective Induction of Human NK Cells with OK-432 and Further Augmentation of Their Cytolytic Function by rIL-2", MICROBIOLOGY AND IMMUNOLOGY, vol. 38, no. 3, 14 March 1994 (1994-03-14) , pages 183-190, XP055090923, ISSN: 0385-5600, DOI: 10.1111/j.1348-0421.1994.tb01763.x
- KUNZMANN, V. ET AL.: 'Stimulation of gammadelta T cells by aminobisphosphonates and induction of antiplasma cell activity in multiple myeloma' BLOOD vol. 96, 2000, pages 384 - 392, XP002223785
- KABELITZ, D. ET AL.: 'Characterization of tumor reactivity of human V gamma 9V delta 2 gamma delta T cells in vitro and in SCID mice in vivo' J. IMMUNOL. vol. 173, no. LL, 2004, pages 6767 - 6776, XP008162495
- JENS KJELDSEN-KRAGH ET AL: 'SELECTIVE ACTIVATION OF RESTING HUMAN GAMMA DELTA T LYMPHOCYTES BY INTERLEUKIN-2' EUROPEAN JOURNAL OF IMMUNOLOGY vol. 23, no. 9, 01 September 1993, pages 2092 - 2099, XP055161201 DOI: 10.1002/eji.1830230908 ISSN: 0014-2980
- A. MANIAR ET AL: 'HUMAN T LYMPHOCYTES INDUCE ROBUST NK CELL-MEDIATED ANTITUMOR CYTOTOXICITY THROUGH CD137 ENGAGEMENT' BLOOD vol. 116, no. 10, 09 September 2010, pages 1726 - 1733, XP055090994 DOI: 10.1182/blood-2009-07-234211 ISSN: 0006-4971

## Description

### Technical Field

The present invention relates to a method for producing a blood product containing activated and grown NK cells, a blood product produced by the method, and a composition for NK cell activation.

### Background Art

Cancer, also known as malignant neoplasm, has been the leading cause of death in Japanese since 1981 and virtually accounts for approximately 30% of all deaths. Although medical advances have drastically improved its cure rate and survival rate, cancer is still an intractable disease. For cancer, surgical therapy, chemotherapy, and radiotherapy are standard treatment methods. In recent years, immunotherapy has received attention as a novel treatment method (Non Patent Literature 1).

The immunotherapy refers to a method for treating cancer, viral infection, or the like by fortifying body's immunity. Particularly, for cancer, the immunotherapy is regarded as a new treatment method comparable to surgical therapy, chemotherapy, and radiotherapy, and various methods have been developed so far. Examples thereof include cytokine therapy, vaccinotherapy, BRM (biological response modifier) therapy, and cellular immunotherapy.

The cytokine therapy refers to a treatment method involving directly administering, into an organism, cytokines having the effect of growing or activating lymphocytes such as T cells or NK cells to thereby kill cancer cells or virus-infected cells. This corresponds to, for example, cytokine therapy based on the administration of interleukin 2 (hereinafter, referred to as "IL-2") (Non Patent Literature 2). Unfortunately, this therapy has failed to produce expected outcomes in clinical trials and causes undesired serious side effect such as organ dysfunction or fluid retention (in the case of IL-2 administration), or cold symptoms or mental disorder (in the case of interferon administration).

The vaccinotherapy refers to a treatment method involving directly inoculating a human body with a cancer cell-specific antigen or the like to activate the immune system against the antigen (Non Patent Literature 3). This treatment method has been reported to be effective for some cases, but is disadvantageously ineffective for tumor or the like without HLA class I expressed therein.

The BRM therapy refers to a treatment method using a substance modifying the biological response of patients to tumor cells or the like (Non Patent Literature 4). For example, PSK, bestatin, and OK-432 are known as BRM. This treatment method, albeit with proven efficacy on some cancers, etc., is more likely to be a supportive therapy that produces effects when used in combination with surgical therapy or other treatment methods such as chemotherapy, which lowers immunity. In addition, this treatment method does not always fortify immunity and, unfortunately, its own anticancer effect or the like is weak.

The cellular immunotherapy refers to a treatment method involving subjecting immunocytes collected from a patient to *ex vivo* treatment such as activation or growth and then bringing these cells back to patient's body to enhance the immunity of the patient, and is also called "adoptive immunotherapy (adoptive immunotherapy in the broad sense)" (Non Patent Literature 5). The cellular immunotherapy is classified into activated lymphocyte therapy and dendritic cell therapy depending on the type of immunocytes treated *ex vivo.* Of them, the dendritic cell therapy has just entered the clinical stage and thus, has not yet produced sufficient results in clinical trials to determine its efficacy.

The activated lymphocyte therapy is further classified into: activated lymphocyte therapy in the narrow sense, which involves activating or growing T cells *ex vivo* (activated T lymphocyte therapy or adoptive immunotherapy in the narrow sense); and activated NK cell therapy, which involves activating or growing NK cells.

The activated lymphocyte therapy in the narrow sense corresponds to, for example, LAK (lymphokine-activated killer cell) therapy, TIL (tumor-infiltrating lymphocyte) therapy, and CTL (cytotoxic T lymphocytes) therapy.

The LAK therapy refers to a method involving adding a large amount of IL-2 to lymphocytes collected from a patient, activating or growing T cells or NK cells by culture, and then bringing these cells back to patient's body (Non Patent Literature 6). This method requires administering a large amount of IL-2 to an organism for maintaining the LAK level administered into the organism, resulting in undesired side effect, as in the IL-2-based cytokine therapy, or less-than-expected effects.

The TIL therapy refers to a method involving collecting lymphocytes infiltrated into tumor cells or the like, culturing them *ex vivo* as in the LAK therapy, and then bringing them back to the body (Non Patent Literature 7). Unfortunately, for this method, surgery is only way to collect lymphocytes, and this method produces less-than-expected effects.

The CTL therapy refers to a method involving stimulating lymphocytes by coculture with cancer cells or the like collected by surgery to induce lymphocytes specific for the cancer cells or the like (Non Patent Literature 8). This method has been reported to be effective for some cases, but is highly invasive and applicable to only limited cases because cancer cells must be collected by surgery. The further problems thereof are, for example: treatment is difficult to achieve if cancer cells can be neither collected nor cultured; and this method is effective only for cancer expressing major histocompatibility antigens.

Meanwhile, the activated NK cell therapy refers to a method involving introducing grown and activated NK cells into the body. NK cells are a population of lymphocytes capable of killing cancer cells or virus-infected cells without being sensitized to antigens (Non Patent Literatures 9 to 11). The NK cells are known to be capable of suppressing cancer infiltration or metastasis in animal experiments (Non Patent Literature 12). According to long-term large-scale cohort study, it has been reported that cancer occurs with a significantly low incidence in humans having highly active NK cells in peripheral blood (Non Patent Literature 13). Hence, NK cells from a patient can be grown and activated in large amounts *ex vivo* and then brought back into patient's body to thereby treat cancer, viral infection, or the like in the patient. The activated NK cell therapy is a treatment method based on such principles. In general, NK cells are found to make up only a small percent to a dozen percent of lymphocytes even in healthy individuals, and the number of NK cells is further reduced in the case of cancer patients. Moreover, NK cells in blood often exhibit lower cytotoxic activity against cancer cells in cancer patients than in healthy individuals even when the same numbers of NK cells are present therein. Thus, growth and activation by culture are absolutely necessary for the therapy. NK cells had been considered difficult to grow *ex vivo.* Nevertheless, many studies have reported in recent years that NK cells were successfully grown and cultured (Non Patent Literatures 14 to 17). These methods, however, utilize cancer cells or transformed cells and have not overcome problems associated with safety in clinical application or practicality. Also, NK cell growth efficiency and cell activity have been at the less-than-satisfactory level. In a further method, azabisphosphonate-capped dendrimers were used for ex-vivo expansion of NK cells (Non Patent Literature 21).

As described above, all the conventional immunotherapy methods have presented insufficient therapeutic effects, serious side effect, or other possible improvements thereto.

Thus, as a result of conducting diligent studies to solve these problems, the inventors of the present application had successfully developed a method for producing an NK cell-enriched blood product, which is capable of efficiently growing and activating NK cells in blood collected from an organism, and received a patent for the production method and the NK cell-enriched blood product (Patent Literature 1). The NK cell-enriched blood product obtained by this method has been used actually in the clinical stage to produce a large number of very favorable clinical outcomes (Non Patent Literatures 18-20). This production method, however, has a slightly complicated production process in which a medium must be kept at a particular temperature for a relatively long time (10 to 30 hours) for the sufficient activation of NK cells. In addition, this method requires laborious temperature control and much time to complete the product.

### Citation List

### Patent Literature

Patent Literature 1: JP Patent No. 4275680

### Non Patent Literature

Non Patent Literature 1: Milani V, et al., 2009, J trans Res, 7 (50): 1-18.
Non Patent Literature 2: Rosenberg SA, et al., 1985, J Exp Med., 161: 1169-88.
Non Patent Literature 3: Bendandi, M. et al., 1999, Nature Med, 5: 1171-1177.
Non Patent Literature 4: Fisher M, et al., 2002, Anticancer Res., 22: 1737-54.
Non Patent Literature 5: Takayama Y et al., 2000, Lancet, 356: 802-807.
Non Patent Literature 6: Mule JJ, et al., 1985, J Immunol., 135: 646-52.
Non Patent Literature 7: Dudley ME, et al., 2003, J Immunother., 26: 332-42.
Non Patent Literature 8: Araki K et al., 2000, Int J Oncol., 17 (6): 1107-18.
Non Patent Literature 9: Stagg J and Smyth M J., 2007, Drug News Perspect, 20 (3): 155-163.
Non Patent Literature 10: Terme M et al., 2008 Nat. Immunol, 9 (5): 486-493.
Non Patent Literature 11: Vivier E et al., 2008, Nat. Immunol, 9 (5): 503-510.
Non Patent Literature 12: DewanMZ et al., 2007, Breast Cancer Res Treat, 104:267-275.
Non Patent Literature 13: Imai K et al., 2000, Lancet, 356: 1795-1799.
Non Patent Literature 14: Harada H et al., 2002, JPN J Cancer Res, 93: 313-9.
Non Patent Literature 15: Carlens S et al., 2001 Hum Immunol, 62: 1092-8.
Non Patent Literature 16: Berg M et al., 2009, Cytotherapy, 11 (3): 341-55.
Non Patent Literature 17: Fujisalci H et al., 2009, Cancer Res, 9: 4010-7.
Non Patent Literature 18: Brillard E et al., 2007, Exp Hematol, 35: 416-425.
Non Patent Literature 19: Cooke A and Brode S., 2008, Critical Rev immununol., 28 (2): 109-126.
Non Patent Literature 20: Hsu KC et al., 2005, Blood, 105: 4878-4884.
Non Patent Literature 21: Portevin et al., 2009, J Translational Med, 7:82

### Summary of Invention

### Technical Problem

An object of the present invention is to develop a novel method for producing an NK cell-enriched blood product, which is less invasive for donors and patients and is capable of rapidly growing and activating NK cells in blood collected from an organism in large amounts by a convenient production process, and to provide an NK cell-enriched blood product obtained by the method in a safe and relatively inexpensive manner.

### Solution to Problem

In order to attain the object, the present inventors have conducted further studies on a method for producing an NK cell-enriched blood product, and consequently successfully developed a novel method which does not require an activation step comprising keeping cells at a particular temperature for a particular time, and which is capable of producing an NK cell-enriched blood product with higher NK cell growth activity than that in the production method according to the prior application (Japanese Patent Application No. 2006-124630; JP Patent No. 4275680) (hereinafter, referred to as the "prior application" in the present specification) for the patented invention of the present inventors.

The present invention has been completed on the basis of these results and provides the following:
(1) A method for producing an NK cell-enriched blood product, comprising: a stimulation step of stimulating NK cells contained in blood collected from an organism, with NK cell growth-stimulating factors comprising at least an anti-CD16 antibody, OK432 (Picibanil), a bisphosphonate derivative or a salt thereof, or a hydrate thereof, and a cytokine; and a culture step of culturing the blood at a physiological cell temperature after the stimulation step, wherein said bisphosphonate derivative is a compound as specified in claim 1.
(2) The method according to (1), wherein the stimulation step comprises keeping the NK cells at 38°C to 40°C for 10 hours to 30 hours to apply thereto high-temperature stimulation.
(3) The method according to (1) or (2), wherein the bisphosphonate derivative is selected from the group consisting of zoledronic acid, pamidronic acid, alendronic acid, risedronic acid, ibandronic acid, incadronic acid, etidronic acid, and a combination thereof.
(4) The method according to any of (1) to (3), wherein the cytokine is IL-2.
(5) The method according to any of (1) to (4), wherein the anti-CD16 antibody is immobilized on a solid-phase support.
(6) The method according to any of (1) to (5), wherein the culture period in the culture step is 9 days to 21 days.
(7) The method according to any of (1) to (5), wherein the blood is peripheral blood.
(8) A composition forNK cell enrichment comprising an anti-CD 16 antibody, OK432, a bisphosphonate derivative or a salt thereof, or a hydrate thereof, and a cytokine, wherein said bisphosphonate derivative is a compound as specified in claim 8.
(9) The composition according to (8), wherein the bisphosphonate derivative is selected from the group consisting of zoledronic acid, pamidronic acid, alendronic acid, risedronic acid, ibandronic acid, incadronic acid, etidronic acid, and a combination thereof.
(10) The composition according to (8) or (9), wherein the cytokine is IL-2.
(11) A kit for production of NK cell-enriched blood comprising an anti-CD16 antibody, OK432, and a bisphosphonate derivative or a salt thereof, or a hydrate thereof, wherein said bisphosphonate derivative is a compound as specified in claim 11.
(12) The kit according to (11), wherein the bisphosphonate derivative is selected from the group consisting of zoledronic acid, pamidronic acid, alendronic acid, risedronic acid, ibandronic acid, incadronic acid, etidronic acid, and a combination thereof.
(13) The kit according to (11) or (12), wherein the cytokine is IL-2.

In a further disclosure, the present specification relates to (8) an NK cell-enriched blood product obtained by a method according to any of (1) to (7).

The present specification encompasses the contents described in the specification and/or drawings of Japanese Patent Application No. 2010-025940 which serves as a basis for the priority of the present application.

### Advantageous Effects of Invention

A method for producing an NK cell-enriched blood product of the present invention can grow NK cells in blood more rapidly and more conveniently at a more improved growth rate than conventional methods. Moreover, the production method of the present invention is capable of producing the product from peripheral blood and is thus advantageously less invasive for donors and patients.

The method for producing an NK cell-enriched blood product of the present invention can enhance not only the growth of NK cells but also the growth of γ-δ T cells, because zoledronic acid is used as one NK cell growth-stimulating factor. As a result, a blood product more effective for cellular immunotherapy can be provided.

The NK cell-enriched blood product of the present invention is free from cytokines or antibiotics as a rule and thus advantageously has no or a very little side effect, unlike conventional cytokine therapy.

### Brief Description of Drawings

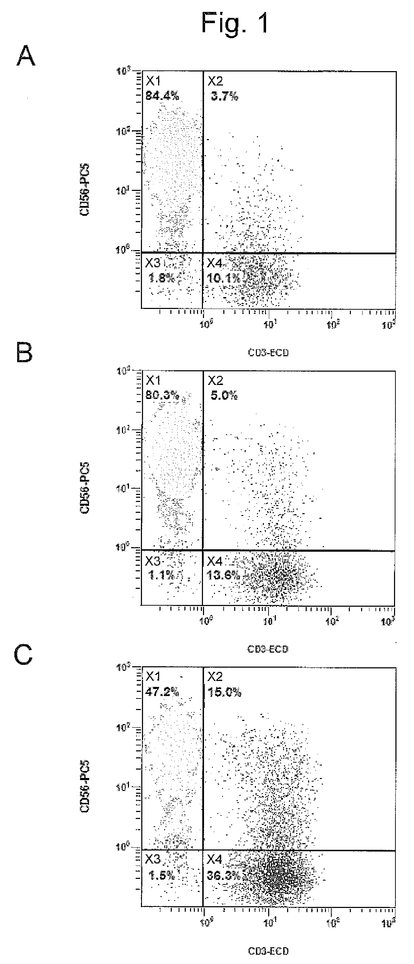

[Figure 1] Figure 1 shows a cytogram at culture day 11 in Example 2 using the fresh blood of a healthy individual. Figures 1A, 1B, and 1C show results about samples A, B, and C, respectively. In this diagram, NK cells are distributed in zone X1; T cells are distributed in zones X2 and X4; and the other cells, such as B cells, are distributed in zone X3.

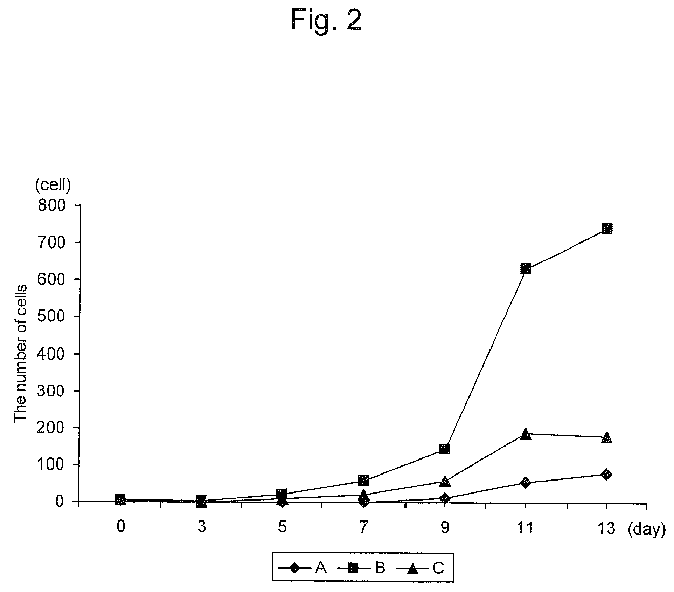

[Figure 2] Figure 2 shows the relationship between the culture time and the number of cultured cells in Example 2 using the fresh blood of a healthy individual. Figures 2A, 2B, and 2C show results about samples A, B, and C, respectively.

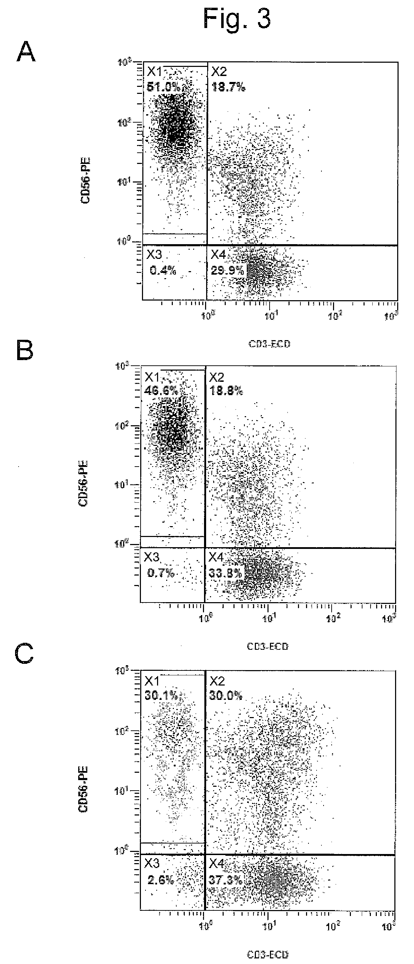

[Figure 3] Figure 3 shows a cytogram at culture day 11 in Example 3 using the frozen blood of a healthy individual. Figures 3A, 3B, and 3C show results about samples A, B, and C, respectively. In this diagram, NK cells are distributed in zone X1; T cells are distributed in zones X2 and X4; and the other cells, such as B cells, are distributed in zone X3.

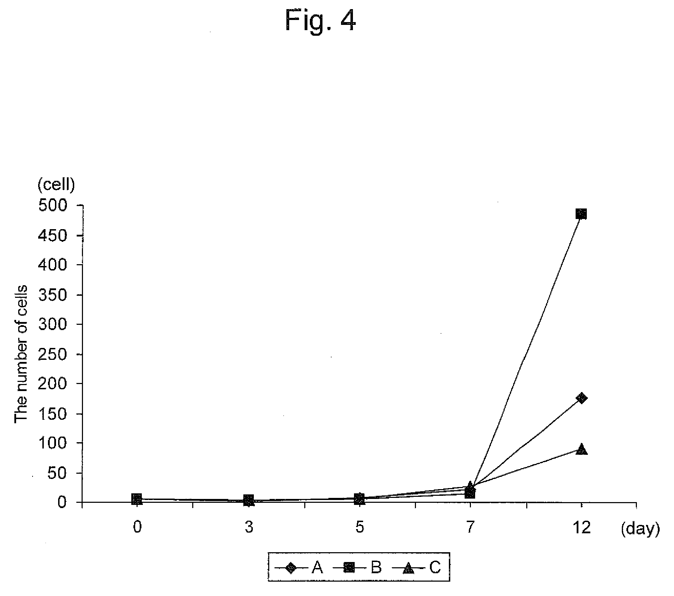

[Figure 4] Figure 4 shows the relationship between the culture time and the number of cultured cells in Example 3 using the frozen blood of a healthy individual. Figures 4A, 4B, and 4C show results about samples A, B, and C, respectively.

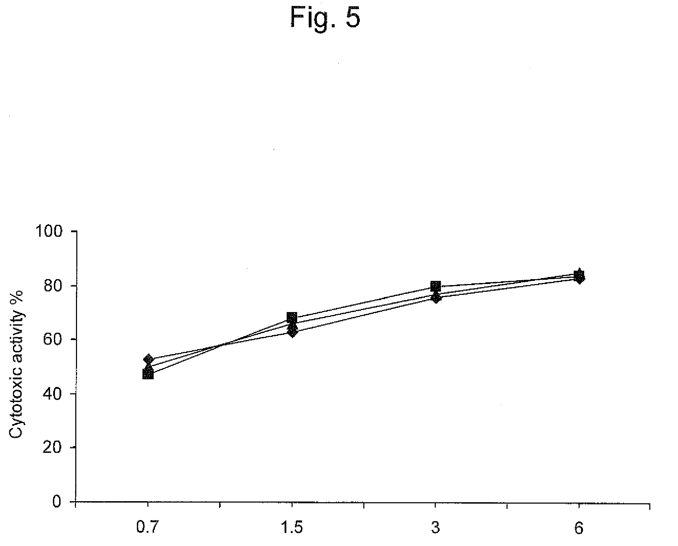

[Figure 5] Figure 5 shows the cytotoxic activity of NK cells against K562.

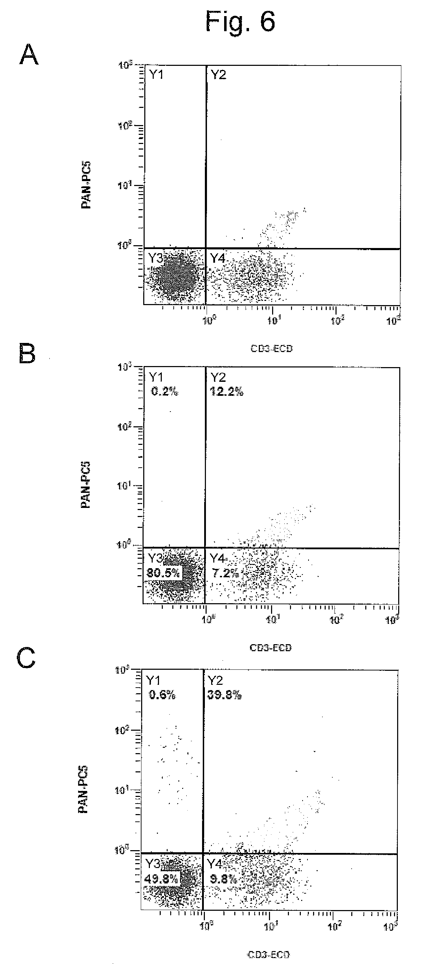

[Figure 6] Figure 6 shows a cytogram at culture day 11 in Example 6 using the fresh blood of a healthy individual. Figures 6A, 6B, and 6C show results about samples A, B, and C, respectively. In this diagram, cells are absent in zone Y1 as a rule; γ-δ T cells are distributed in zone Y2; cells (including NK cells) other than T cells are distributed in zone Y3; and α-β T cells are distributed in zone Y4.

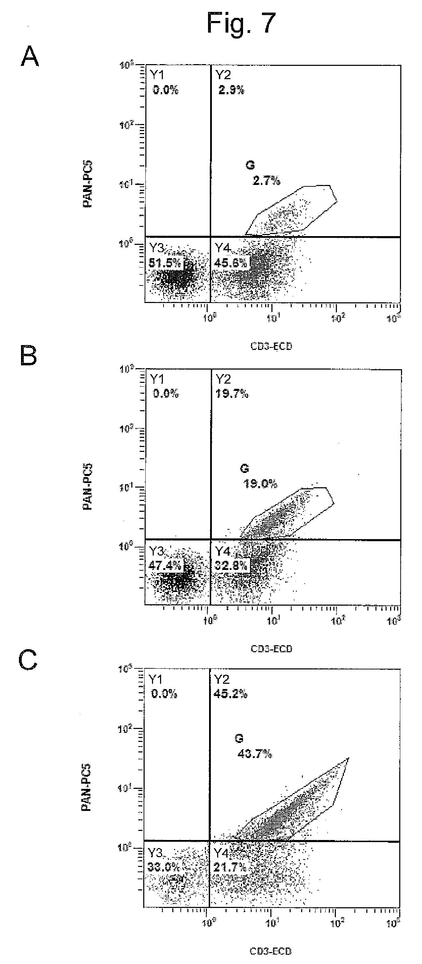

[Figure 7] Figure 7 shows a cytogram at culture day 11 in Example 6 using the frozen blood of a healthy individual. Figures 7A, 7B, and 7C show results about samples A, B, and C, respectively. In this diagram, cells are absent in zone Y1 as a rule; γ-δ T cells are distributed in zone Y2; cells (including NK cells) other than T cells are distributed in zone Y3; and α-β T cells are distributed in zone Y4.

### Description of Embodiments

### 1. Method for producing NK cell-enriched blood product

### 1-1. Summary

A first embodiment of the present invention relates to a method for producing an NK cell-enriched blood product according to claim 1. The feature of this embodiment is that NK cells in blood collected from an organism are stimulated with growth-stimulating factors, and then, the blood is cultured at a physiological cell temperature.

In the present invention, the "NK cell-enriched blood product" refers to a product mainly composed of blood containing a large number of activated NK cells obtained by the production method of this embodiment. In the present invention, the term "enrichment" means to grow and activate cells or to have grown and activated cells. In the present specification, the term "activation" means to enhance or potentiate functions possessed by cells, particularly, NK cells. Examples thereof include to potentiate cytotoxic function and to potentiate the expression of NK cell surface receptors involved in activity and/or growth. Thus, in the present specification, the activation of cell growth is indicated by "growth activity (activation)" aside from the term "activation" described above. The NK cell-enriched blood product of the present invention incorporates the number of NK cells approximately 100 or more times that in the blood of a healthy individual per unit volume.

### 1-2. Constitution

The method for producing an NK cell-enriched blood product of this embodiment comprises a stimulation step and a culture step. Hereinafter, the constitution of each step will be described specifically. This embodiment is based on the premise that, as a rule, each operation employs already sterilized reagents, media, tools, etc., and culture is performed in a sterile environment such as a clean bench in a clean room. This is because blood and the NK cell-enriched blood product produced in this embodiment are prevented from being contaminated with bacteria or the like.

### 1-2-1. Stimulation step

The "stimulation step" is a step of stimulating NK cells contained in blood collected from an organism with NK cell growth-stimulating factors.

### (1) Blood

In the present invention, the "blood" refers to a blood component containing at least NK cells. The blood (component) corresponds to, for example, whole blood, cord blood, bone marrow fluid, or a portion of its components, for example, mononuclear cells. Mononuclear cells are preferable. This is because blood components such as erythrocytes or granulocytes might become a hindrance to the production of the NK cell-enriched blood product. Peripheral blood mononuclear cells (PBMCs) obtained from peripheral blood are particularly preferable. This is because, for example, peripheral blood can be collected easily from organisms at any time with procedures much less invasive for the donors.

In the present invention, the "organism" refers to a living mammal. The type of the mammal is not particularly limited, and a human is preferable. The donor organism is of the same type as in an animal that receives the NK cell-enriched blood product obtained by the production method of the present invention. For example, when the NK cell-enriched blood product of the present invention is administered to a human, blood is collected from a human. Most preferably, blood is collected from an organism itself that receives the NK cell-enriched blood product of the present invention, i.e., blood collection is predicated on adoptive immunotherapy. This is because a blood product derived from recipient's own blood can minimize the possibility of rejection af ter administration. When blood collection is predicated on adoptive immunotherapy, the donor organism does not have to be healthy. For example, blood can be collected even from a patient having cancer or viral infection. In the present invention, the adoptive immunotherapy in the description below means the adoptive immunotherapy in the broad sense described above, unless otherwise specified.

The phrase "collected from an organism" means derived from an organism. Thus, this phrase encompasses any of blood directly collected from the organism and blood indirectly collected therefrom. The directly collected blood corresponds to, for example, peripheral blood or bone marrow fluid collected by the direct insertion of an injection needle or the like to the organism, or, for example, cord blood collected directly from the postpartum umbilical cord. Alternatively, the indirectly collected blood refers to, for example, blood obtained by adding heparin or the like for anticoagulation treatment to the directly collected blood or further isolating mononuclear cells therefrom and then temporarily refrigerating or cryopreserving it, followed by collection.

### (2) Preparation of blood

When the blood used in this step is directly collected from the organism, a collection method therefor can follow a blood collection method known in the art. For example, peripheral blood may be collected by injection to the peripheral vein or the like; bone marrow fluid may be collected by bone marrow aspiration; and cord blood may be collected by the injection of a needle to the postpartum umbilical cord before placenta delivery. Hereinafter, the collection of peripheral blood will be described specifically.

Peripheral whole blood can be collected into a syringe by the insertion of an injection needle to the peripheral blood vessel, for example, the vein or artery, of the organism. The volume of blood collected varies depending on the necessary amount of the NK cell-enriched blood product. Usually, 20 mL to 60 mL suffices for the blood product produced, for example, for a single dose to a human adult. However, the number of peripheral blood mononuclear cells in blood may be extremely reduced, for example, in cancer patients. In such a case, only peripheral blood mononuclear cells may be collected selectively in a necessary amount by apheresis. For preventing the coagulation of the blood thus collected, it is preferred to coat in advance, for example, the inside of a syringe, with an anticoagulant such as heparin or a blood coagulation inhibitor, or to add heparin or the like to the collected blood. Alternatively, plasma is separated from the peripheral whole blood, and the remaining blood cell components may be used. The plasma can be obtained as a supernatant, for example, by the centrifugation at 2000 rpm to 4000 rpm for 5 to 20 minutes of peripheral whole blood transferred to a centrifuge tube. Furthermore, this supernatant can be inactivated by heating at 56°C for approximately 30 minutes, further centrifuged at 2000 rpm to 4000 rpm for 5 to 20 minutes, and used as nutrients for cell culture after removal of precipitates such as platelet.

Peripheral blood mononuclear cells (PBMCs) may be further separated, if necessary, from the peripheral whole blood. PBMCs can be obtained from peripheral whole blood or from blood cell components after plasma separation using a density-gradient centrifugation method with Ficoll-Hypaque or Ficoll-Conray as a specific gravity solution. A commercially available separating solution or the like can be used conveniently as such a specific gravity solution. For example, Ficoll-Paque PLUS (Amersham Biosciences) or LYMPHOPREP (AXIS-SHIELD plc) can be used. A method for separating PBMCs can follow the protocol supplied with the kit.

The PBMCs thus separated are washed several times with PBS (-) or a medium for cultured cells to remove the specific gravity solution. In this context, for example, serum-free PBS (-), a RPMI1640 medium, or a serum-free medium for use in culture can be used as the medium for cultured cells. After the washing with PBS (-) or such a medium, it is preferred to count the number of collected PBMCs using a hemacytometer. Usually, 2 × 10⁷ or more PBMCs can be collected from 20 mL to 60 mL of peripheral whole blood.

When the blood used in this step is indirectly collected from the organism, a collection method therefor can involve thawing or heating frozen or refrigerated blood for use by a method known in the art. Examples thereof include a method involving adding a RPMI-1640 medium, for thawing, to the PBMCs obtained from the directly collected blood and then cryopreserved, and then incubating the thawed PBMCs at 37°C for 3 hours under 5% CO₂ condition.

### (3) NK cell growth-stimulating factor

In the present invention, the "NK cell growth-stimulating factor" refers to a factor directly or indirectly inducing the growth and/or activation of NK cells. Examples of the factor directly inducing the growth include factors having the function of transmitting growth signals into NK cells through the specific binding to the surface receptors of the NK cells to be grown. Examples of the factor indirectly inducing the growth include factors inducing the production and release of liquid factors such as cytokines through the binding to the surface receptors of cells other than NK cells, such as monocytes. In this case, the growth of NK cells is indirectly induced by the released liquid factor. Examples of the factor directly or indirectly inducing the activation of NK cells include factors similar in mechanism to the factors inducing the growth.

The NK cell growth-stimulating factors of the present invention comprises at least an anti-CD16 antibody, OK432, a bisphosphonate derivative or a salt thereof, or a hydrate thereof, and a cytokine. In addition, the NK cell growth-stimulating factors may comprise, for example, BRM such as Lentinan extracted from shiitake mushrooms.

The "anti-CD16 antibody" refers to an antibody against CD16. CD16 is a marker for NK cells or granulocytes and is known as a protein constituting Fc receptor present on the surfaces of most NK cells. The NK cell growth-inducing activity of the anti-CD16 antibody was found by the prior patent application and had been unknown before then. Although the mechanism underlying the induction of NK cell growth by the anti-CD16 antibody remains to be elucidated, the co-addition of the anti-CD16 antibody and a cytokine such as IL-2 can drastically potentiate the induction of NK cell growth compared with the addition of the cytokine alone (Japanese Patent Application No. 2006-124630; JP Patent No. 4275680).

The "bisphosphonate derivative" refers to a compound represented by the following general formula 1:

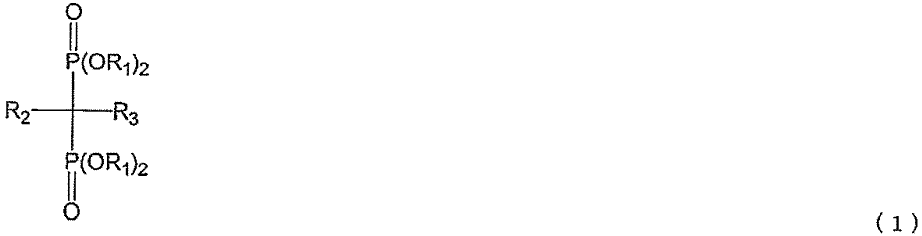

In the formula, R₁ represents a hydrogen atom (H) or a lower alkyl group; R₂ and R₃ each independently represent a hydrogen atom, halogen, a hydroxyl group, an amino group, a thiol group, a substituted or unsubstituted aryl group, a substituted or unsubstituted alkyl group, a lower alkylamino group, an aralkyl group, a cycloalkyl group, or a heterocyclic group, or R₂ and R₃ form a portion of a cyclic structure containing them wherein substituents forming the cyclic structure are each independently derived from halogen, a lower alkyl group, a hydroxyl group, a thiol group, an amino group, an alkoxy group, an aryl group, an arylthio group, an aryloxy group, an alkylthio group, a cycloalkyl group, or a heterocyclic group in R₂ and R₃.

Specific examples of the bisphosphonate derivative include zoledronic acid, pamidronic acid, alendronic acid, risedronic acid, ibandronic acid, incadronic acid, and etidronic acid. In the present invention, one or more bisphosphonate derivatives or salts thereof, or hydrates thereof can be added as the NK cell growth-stimulating factor. In the present invention, the bisphosphonate derivative is particularly preferably zoledronic acid or a zoledronic acid derivative capable of inducing the growth and/or activation of NK cells (hereinafter, referred to as "growth/activation") or a salt thereof, or a hydrate thereof.

The zoledronic acid (trade name: Zometa (registered trademark), Novartis Pharma K.K.) is bisphosphonate having bone resorption inhibitory activity and is known as a therapeutic drug for hypercalcemia caused by malignant tumor, bone lesions attributed to multiple myeloma, and bone lesions attributed to solid cancer metastasized to bone. Since, its chemical structure incorporates nitrogen-containing bisphosphonates (N-BPs), this acid inhibits the intracellular synthesis of farnesyl pyrophosphate (FPP), resulting in the accumulation of its precursor isopentenyl pyrophosphate (IPP). As a result, the immune response of the organism can reportedly be activated (van Beek E, et al., 1999, Biochem Biophys Res Commun, 264: 108-11; and Gober HJ, et al., 2003, J Exp Med, 197: 163-8). However, it has not been reported by any means that zoledronic acid induces the growth/activation of NK cells. Thus, the zoledronic acid is a novel NK cell growth-stimulating factor that has been found by the present inventors for the first time.

The "salt thereof" refers to a base-addition salt of the bisphosphonate derivative, preferably zoledronic acid. Examples of the base-addition salt include: alkali metal salts such as sodium salt and potassium salt; alkaline earth metal salts such as calcium salt and magnesium salt; aliphatic amine salts such as trimethylamine salt, triethylamine salt, dicyclohexylamine salt, ethanolamine salt, diethanolamine salt, triethanolamine salt, and procaine salt; aralkylamine salts such as N,N-dibenzylethylenediamine; heterocyclic aromatic amine salts such as pyridine salt, picoline salt, quinoline salt, and isoquinoline salt; basic amino acid salts such as arginine salt and lysine salt; and ammonium salt and quaternary ammonium salts such as tetramethylammonium salt, tetraethylammonium salt, benzyltrimethylammonium salt, benzyltriethylammonium salt, benzyltributylammonium salt, methyltrioctylammonium salt, and tetrabutylammonium salt.

The "OK432" (Picibanil) refers to an antitumor agent comprising a penicillin-treated Su strain of hemolytic streptococcus (type III group A *Streptococcus pyogenes*) as an active ingredient. OK432 is known to serve as an immune adjuvant capable of activating, for example, monocytes, through the binding to the surface TLR of the monocytes so that immune response is activated (Ryoma Y, et al., 2004, Anticancer Res., 24: 3295-301).

Examples of the cytokine appropriate for the NK cell growth-stimulating factor of the present invention include IL-2, IL-12, IL-15, and IL-18. Of them, IL-2 is particularly preferable as the cytokine.

### (4) Method for applying stimulation

The term "stimulating" refers to contacting the NK cell growth-stimulating factors with NK cells to thereby induce the growth/activation of the NK cells.

In a specific method for applying stimulation thereto, first, blood, for example, PBMCs, collected from an organism is adjusted with a medium into, for example, a cell density of 1 to 3 × 10⁶ cells/mL. In this context, the medium used may be any appropriate medium for cell culture supplemented with inactivated human serum or plasma at a volume ratio (V/V) on the order of 5 to 10%. When the blood product is predicated on administration for the adoptive immunotherapy, it is preferred to use, as the medium, a serum-free medium for adoptive immunotherapy such as OpTmizer supplemented with autologous plasma. The autologous plasma can be prepared from blood obtained after the blood collection step. For example, the collected peripheral whole blood is centrifuged at 3000 rpm at room temperature (10°C to 30°C: the same holds true for the description below) for approximately 10 minutes to obtain a supernatant, which can in turn be used as the autologous plasma. If necessary, the medium may be further supplemented with an antibiotic such as streptomycin, penicillin, kanamycin, or gentamicin.

Next, each NK cell growth-stimulating factor is added to the culture solution containing the preliminarily prepared PBMCs.

For stimulation with the anti-CD16 antibody, this antibody can be added directly to the medium at, for example, 50 µg/mL to 200 µg/mL, preferably 70 µg/mL to 150 µg/mL, more preferably 100 µg/mL, in terms of the final concentration, or can be added thereto in a form immobilized on a solid-phase support. The addition in a form immobilized on a solid-phase support is preferable. This is because the anti-CD16 antibody thus immobilized on a solid phase can come into contact with NK cells with increased frequency in the constant direction and thus efficiently impart growth stimulation to the NK cells compared with a free form. In this context, the "support" refers to a scaffold for antibody immobilization. A material for the support is not particularly limited as long as this material permits stable immobilization of the antibody. For example, a synthetic resin (e.g., plastic), glass, or metal can be used. The shape of the support is not particularly limited. A shape with a large surface area of contact with the culture solution is preferable because the antibody immobilized on this support can come into contact with the NK cells with higher frequency. Examples thereof include spherical beads and porous cubes having lymphocyte-sized pores.

When the support is made of a material with high affinity for the antibody, for example, plastic, the anti-CD16 antibody can be immobilized on this support by a simple method of contacting (including dipping, coating, circulating, spraying, etc.) the antibody solution with the support and keeping them at a predetermined temperature for a predetermined time. The anti-CD16 antibody solution can be obtained, for example, by dissolving the anti-CD16 antibody in sterile distilled water or a medium for cell culture, then sterilizing, if necessary, by filtration through, for example, a filter of 0.22 µm in pore size, and adjusting the filtrate with sterile distilled water or a medium to 1 µg/mL in terms of the final concentration. For immobilizing the anti-CD16 antibody on the support, it is preferred to use the anti-CD16 antibody solution in a volume in consideration of the surface area or the like of the solid-phase support. For example, approximately 15 mL of 1 µg/mL anti-CD16 antibody solution can be used for immobilization on a plastic flask having an inner wall surface area of 150 cm². Alternatively, 5 mL and 10 mL thereof can be used for culture flasks having an inner wall surface area of 25 cm² and 75 cm², respectively. The anti-CD16 antibody is attached to the support by subsequent incubation at 37°C for 12 to 24 hours. Alternatively, a commercially available antibody immobilization kit or the like may be used. For example, CarboLink (Pierce Biotechnology, Inc.) can be used. Such an immobilization kit is useful for supports made of a material difficult to attach to antibodies.

After the immobilization of the anti-CD 16 antibody on the support, the support with the anti-CD16 antibody immobilized thereon can be washed, if necessary, to remove the anti-CD16 antibody solution. For example, the support can be washed several times, for example, approximately 2 to 5 times, with an appropriate amount of PBS. Such a culture container comprising the anti-CD16 antibody thus immobilized on the support can be stored at 0°C to 8°C, preferably 3°C to 6°C and thereby used for approximately 1 month without reducing or inactivating the avidity of the antibody.

Hereinafter, stimulation with the bisphosphonate derivative or a salt thereof, or a hydrate thereof will be described specifically by taking a case using 4 mg/vial of a zoledronic acid hydrate injection (Novartis Pharma K.K.) as an example. This zoledronic acid is dissolved in water (e.g., injectable water) to prepare a 2.94 µmol/mL zoledronic acid solution. If necessary, the solution may then be sterilized by filtration through, for example, a filter of 0.22 µm in pore size. The obtained zoledronic acid solution can be added to the medium at, for example, 1 µM/mL to 10 µM/mL, preferably 3 µM/mL to 7 µM/mL, more preferably 5 µM/mL, in terms of the final concentration.

For stimulation with OK432, for example, an OK432 solution can be added at, for example, 0.005 KE/mL to 0.02 KE/mL, preferably 0.008 KE/mL to 0.015 KE/mL, more preferably 0.01KE/mL, in terms of the final concentration to the culture solution containing PBMCs. The OK432 solution can be prepared by dissolving Picibanil (5 KE/vial) from Chugai Pharmaceutical Co., Ltd. in 2 mL of water (e.g., injectable water).

For stimulation with the cytokine, one type or a combination of several types can be added thereto. It consideration of cost, etc., it is preferred to add only IL-2. The amount of, for example, IL-2, added is preferably in the range of 100 units/mL to 2000 units/mL, in terms of the final concentration. This is because: an amount smaller than 100 units/mL is insufficient for inducing growth stimulation; and an amount larger than 2000 units/mL does not offer the growth of NK cells according to increase in IL-2 concentration. This amount is preferably in the range of 700 units/mL to 2000 units/mL.

For sufficiently stimulating PBMCs, it is preferred to keep the blood at a physiological cell temperature (usually, 37°C) for 1 to 3 days after the addition of each of these NK cell growth-stimulating factors. In this period, which may overlap with the period of the subsequent culture step, the NK cells, etc. can be cultured with stimulation applied thereto.

During the period for which the blood is kept at a physiological cell temperature, high-temperature stimulation may be applied thereto at 38°C to 40°C for a period of 10 hours to 30 hours. This high-temperature stimulation can further activate the NK cells. This is because, as shown in Examples of the prior application, a temperature lower than 37°C is insufficient for activating lymphocytes; and a temperature higher than 40°C makes lymphocytes more likely to be degenerated or damaged by heat. This is also because: a time shorter than 10 hours is insufficient for further activation; and a time longer than 30 hours makes blood more likely to be degenerated or damaged.

Means of keeping the blood at a predetermined temperature is not particularly limited as long as the blood can be kept at the constant temperature by this means. Examples thereof include means by which the blood together with its container is kept at a predetermined temperature using a CO₂ incubator.

### 1-2-2. Culture step

The "culture step" is the step of culturing the blood at a physiological cell temperature after the stimulation step. The feature of this step is that the number of the NK cells is increased with their growth/activation maintained.

The "physiological cell temperature" refers to the optimum temperature for culturing the blood. This temperature is usually 37°C and may be the temperature with a tolerance of less than ±0.5°C. This is because the internal temperature of a thermostat might fluctuate within this temperature range.

This step can be achieved by culturing the culture solution that has undergone the stimulation step, at a physiological cell temperature. At the initial stage of this step, the period for which the NK cells are sufficiently stimulated with the NK cell growth-stimulating factors added in the stimulation step can be secured and also serve as the period for which these cells are cultured. Then, it is preferred to temporarily remove the NK cell growth-stimulating factors from the medium to thereby cancel the stimulation step. This is because, although most of factors such as cytokines can impart growth/activation-inducting stimulation to the NK cells even in the culture step, the long-term stimulation of the NK cells with the anti-CD16 antibody, OK432, and/or zoledronic acid or the like might have undesired influence, for example, cell death (e.g., apoptosis), on NK cell enrichment. Such stimulating factors can be removed by a method involving, for example, temporarily collecting PBMCs from the culture solution after the stimulation step and then transferring these PBMCs to a new culture solution free from the anti-CD16 antibody, OK432, and zoledronic acid or the like. The removal of the factors is achieved by centrifuging the blood that has undergone the stimulation step and removing the supernatant. Its specific method can follow a medium replacement method described below.

The culture is performed for a period from 7 days to 21 days in a 5% CO₂ incubator that satisfies the physiological cell temperature condition. This is because: 6-day or shorter culture is insufficient for the growth of NK cells; and in 22-day or longer culture, the growth of NK cells reaches the resting phase or such long-term culture makes cells more likely to be damaged (e.g., deteriorated (aged)).

For the culture period, it is preferred to add a fresh medium or replace the medium by a fresh medium at regular intervals of 2 days to 5 days. As a specific example of the medium replacement, first, the culture solution containing PBMCs after the stimulation step is transferred to an already sterilized centrifuge tube. Subsequently, the tube is centrifuged at approximately 1200 rpm at room temperature for approximately 8 minutes, and the supernatant is then removed to collect PBMCs or NK cells as precipitates. The collected PBMCs or NK cells are transferred at a cell density of 0.6 to 1.0 × 10⁶ cells/mL to a fresh culture solution containing IL-2 and plasma. In this context, the cytokine such as IL-2 can be added thereto at approximately 300 units/mL to approximately 700 units/mL in terms of the final concentration. This is because the NK cells have already been activated after the stimulation step and produce cytokines such as IL-2 in themselves.

The medium used in the culture can be any general medium for use in cell culture as a rule. OpTmizer medium (Life Technologies Corp.) is preferable.

After the culture, the culture solution is confirmed to have no contamination with bacteria or endotoxin. The presence or absence of bacteria can be examined by colony formation assay, while the presence or absence of endotoxin can be examined by colorimetry such as commercially available ELISA or by a suspension method such as limulus test.

### 1-3. Effect

The method for producing an NK cell-enriched blood product of this embodiment can produce an NK cell-enriched blood product from blood collected from an organism. Particularly, the production method can provide an NK cell-enriched blood product containing NK cells which has the activity as high as that obtained in the method for producing an NK cell-enriched blood product of the prior application, and which has been grown at a rate exceeding the high growth rate of the prior application.

According to the method for producing an NK cell-enriched blood product of this embodiment, the essential step of keeping blood at a predetermined temperature for a predetermined time (activation step; which corresponds to high-temperature stimulation in the stimulation step according to the present invention) in the prior application is no longer essential. As a result, an incubator set to a predetermined temperature necessary for the activation step is not necessarily required. This can drastically reduce burdens from an equipment standpoint in research facilities where the present invention is carried out, or burdens in terms of operation/management by an operator.

According to the production method, γ-δ T cells can also be grown at the same time with NK cells using zoledronic acid, as shown in Example 6 described later. The γ-δ T cells are also effective for cellular immunotherapy, as in NK cells. Thus, the production method can produce synergistic effect, i.e., the growth of NK cells and γ-δ T cells, which has not been achieved by the production of the prior application.

Moreover, the production method can minimize physical burdens on donors because the blood collected from an organism may be peripheral blood.

The production method does not require particular special equipment or the like and can utilize regular equipment or the like installed in general testing facilities, research facilities, etc., for cell culture. In addition, any of necessary reagents, etc. can be obtained easily. Accordingly, the production method of this embodiment can be carried out advantageously in research facilities capable of aseptic manipulation, such as clean room, without the need of initial equipment investment or the like.

The NK cell-enriched blood product obtained by the production method of this embodiment has the effect of preventing the recurrence of cancer or blocking the progression of cancer in actual clinical treatment. In addition, the blood product that can be provided is safe in such a way that the administration of the blood product has been confirmed to have no side effect.

### 2. NK cell-enriched blood product

### 2-1. Summary

A further disclosure of the present specification relates to an NK cell-enriched blood product obtained by the production method of the first embodiment.

### 2-2. Constitution

The NK cell-enriched blood product of this disclosure can be obtained from the culture solution that has undergone the culture step in the first embodiment. However, the NK cell-enriched blood product does not require the medium used in the culture or the growth-stimulating factors added to the medium. Thus, for use of the NK cell-enriched blood product, it is preferred to remove the medium and the growth-stimulating factors as much as possible from the culture solution to obtain grown/activated NK cells, etc. The medium and the growth-stimulating factors are removed by a method involving first transferring the culture solution containing the grown/activated NK cells to an already sterilized centrifuge tube, followed by centrifugation at 1200 rpm at room temperature for approximately 8 minutes to remove the medium in a supernatant containing the growth-stimulating factors. The NK cells can be collected as precipitates. It is preferred to wash the collected NK cells two or more times with PBS (-). The number of the NK cells thus washed is counted using a hemacytometer and adjusted with 10 mL to 200 mL of a lactate Ringer solution or saline. In this way, the NK cell-enriched blood product of this embodiment can be obtained. If necessary, cytokines or the like may be added to the blood product.

For obtaining sufficient effects using the blood product of this disclosure, it is preferred that the NK cells contained therein should have a cytotoxic activity of 70% or higher against cancer cells. The cytotoxic activity of the NK cells against cancer cells can be determined by assay known in the art using a leukemia cell line K562. Alternatively, a marker known in the art such as a lymphocyte activation marker, for example, CD69, can be used.

### 2-3. Effect

According to the NK cell-enriched blood product of this disclosure, an NK cell-enriched blood product containing activated NK cells as many as 5 × 10⁷ to 4 × 10⁹ cells can be obtained from 20 mL to 60 mL of peripheral whole blood.

In the NK cell-enriched blood product of this disclosure, approximately 90% of the NK cells are active. This corresponds to 10 or more times that in the blood of a healthy adult.

The NK cell-enriched blood product of this disclosure may be used immediately after its production or may be stored either for a predetermined period at a temperature of 0°C to 8°C or for a period as long as several years at a ultralow temperature (approximately -80°C) or in liquid nitrogen after being supplemented with a storage solution or the like. A commercially available activated lymphocyte storage solution can be used conveniently as the storage solution. For example, Bambanker (Nippon Genetics Co., Ltd.) or KM Banker II (Cosmo Bio Co., Ltd.) can be used.

### 3. Composition for NK cell enrichment

### 3-1. Summary

The second embodiment of the present invention relates to a composition for NK cell enrichment according to claim 8. The feature of this embodiment is that the composition comprises the NK cell growth-stimulating factors comprising the bisphosphonate derivative or the salt thereof, or the hydrate thereof, particularly, zoledronic acid, which has been newly found by the present invention. The composition for NK cell enrichment of this embodiment can be added to blood, preferably a medium containing PBMCs, to thereby conveniently and efficiently growing/activating NK cells in the medium.

### 3-2. Constitution

The "composition for NK cell enrichment" refers to a composition capable of growing/activating NK cells presented in a medium through its addition to the medium.

The composition for NK cell enrichment of this embodiment comprises the anti-CD16 antibody, the OK432, the bisphosphonate derivative represented by the formula 1 or the salt thereof, or the hydrate thereof, and the cytokine described in the first embodiment. The bisphosphonate derivative is preferably a compound selected from the group consisting of zoledronic acid, pamidronic acid, alendronic acid, risedronic acid, ibandronic acid, incadronic acid, and etidronic acid, more preferably, zoledronic acid. The cytokine is preferably a compound selected from the group consisting of IL-2, IL-12, IL-15, and IL-18, more preferably IL-2. The composition for NK cell enrichment may additionally incorporate medium components for lymphocytes, such as RPMI-1640, a pH stabilizer, an antibiotic, etc.

These components in the composition can be mixed in amounts that give their respective predetermined final concentrations when added to a predetermined amount of a medium. Specifically, these components can be mixed so that: the anti-CD16 antibody gives a final concentration of 50 µg/mL to 200 µg/mL, preferably 70 µg/mL to 150 µg/mL, more preferably 100 µg/mL; the bisphosphonate derivative or the salt thereof, or the hydrate thereof (preferably, zoledronic acid) gives a final concentration of 1 µM/mL to 10 µM/mL, preferably 3 µM/mL to 7 µM/mL, more preferably 5 µM/mL; the OK432 gives a final concentration of 0.005 KE/mL to 0.02 KE/mL, preferably 0.008 KE/mL to 0.015 KE/mL, more preferably 0.01 KE/mL; and the cytokine (preferably, IL-2) gives a final concentration of 500 U/mL to 2000 U/mL, preferably 700 U/mL to 1500 U/mL, more preferably 1000 U/mL.

The dosage form of the composition is not particularly limited. The composition can be in a liquid form dissolved in an appropriate buffer, a powdery form, or in the form of tablets prepared from a powder supplemented with an appropriate excipient, etc. Alternatively, the composition may be a mixture of different forms and is, for example, in a dosage form in which the anti-CD16 antibody immobilized on a solid-phase support such as plastic beads is mixed with a solution containing the OK432, the bisphosphonate derivative represented by the formula 1 or the salt thereof, or the hydrate thereof, and the cytokine dissolved in a liquid.

### 3-3. Effect

According to the composition for NK cell enrichment of this embodiment, NK cells can be activated and grown by simple procedures of addition to a predetermined amount of an appropriate cell culture solution containing the NK cells and culture.

### 4. Kit for production of NK cell-enriched blood

### 4-1. Summary

A third embodiment of the present invention relates to a kit for production of NK cell-enriched blood comprising the anti-CD16 antibody, the OK432, the bisphosphonate derivative represented by the formula 1 or the salt thereof, or the hydrate thereof, and the cytokine described in the first embodiment. This embodiment incorporates, as components, the NK cell growth-stimulating factors comprising the bisphosphonate derivative or the salt thereof, or the hydrate thereof, particularly, zoledronic acid, which has been newly found by the present invention. This kit can be used in the culture of blood, preferably PBMCs, to thereby conveniently and easily produce an NK cell-enriched blood product.

### 4-2. Constitution

The kit for production of NK cell-enriched blood of this embodiment comprises the anti-CD16 antibody, the OK432, the bisphosphonate derivative represented by the formula 1 or the salt thereof, or the hydrate thereof, and the cytokine described in the first embodiment. The kit for production of NK cell-enriched blood may additionally incorporate sterile water or buffers for dissolving each NK cell growth-stimulating factor, an instruction manual, etc. The bisphosphonate derivative is preferably a compound selected from the group consisting of zoledronic acid, pamidronic acid, alendronic acid, risedronic acid, ibandronic acid, incadronic acid, and etidronic acid, more preferably zoledronic acid. The cytokine is preferably a compound selected from the group consisting of IL-2, IL-12, IL-15, and IL-18, more preferably IL-2.

These NK cell growth-stimulating factors can be incorporated alone or in combination of two or more thereof in the kit. For example, the NK cell growth-stimulating factors other than the anti-CD16 antibody may be incorporated in one portion in the kit. The state of each NK cell growth-stimulating factor is not particularly limited. One NK cell growth-stimulating factor may be in a liquid state while the other NK cell growth-stimulating factors may be in a solid state. Particularly, it is preferred that the anti-CD16 antibody should be incorporated therein in a form immobilized on an appropriate solid-phase support such as plastic beads.

### 5. Cellular immunotherapy to treat disease

### 5-1. Summary

A further disclosure of the present specification relates to cellular immunotherapy for treating a disease, involving administering the NK cell-enriched blood product produced in the first embodiment to an organism to enhance its immunity.

### 5-2. Constitution

This disclosure relates to cellular immunotherapy involving administering the NK cell-enriched blood product obtained by the production method of the first embodiment to an organism.

The "cellular immunotherapy" according to this disclosure refers to a method for treating a disease, involving administering the NK cell-enriched blood product obtained by the production method of the first embodiment to an organism to enhance the immunity of the organism. Particularly, for the cellular immunotherapy of this disclosure, it is preferred to be predicated on adoptive immunotherapy. This is because the adoptive immunotherapy is substantially free from the risk of rejection, as described above.

The NK cell-enriched blood product administered thereto contains a larger number of lymphocytes having immunity against cancer, viral infection, or fungal infection than the average number thereof in usual blood per unit volume. In this context, the "cancer" means general malignant tumor. The cancer corresponds to, for example, epithelial tumor, sarcoma, leukemia, and myeloma. The "viral infection" refers to general disease caused by infection with a virus and particularly corresponds to the prevention of intractable chronic viral infection and acute viral infection. Examples of the intractable chronic viral infection include HIV infection causative of AIDS, chronic viral hepatitis, and human papillomavirus infection causative of uterine cervix cancer. Examples of the acute viral infection include viral respiratory infection such as influenza, and acute viral infection in an immunodeficient state. The "fungal infection" refers to infection with filamentous bacteria, yeast, or the like. Examples thereof include candidal infection, blastomycosis, and histoplasmosis.

The "lymphocyte having immunity" means a lymphocyte having fortified functions in the immune system. Such lymphocytes correspond to, for example, NK cells, γ-δ T cells, killer T cells, and NKT cells that have been activated to be cytotoxic. In this context, the "average value in usual blood per unit volume" means the average number per unit volume of blood cells having immunity against cancer, viral infection, or fungal infection generally observed in the blood of healthy individuals. For example, approximately 5 × 10⁵ NK cells on average are present per mL of blood of a healthy adult individual. The feature of the NK cell-enriched blood product used in this disclosure is that the number of any one or two or more of lymphocytes having immunity against cancer, viral infection, or fungal infection, for example, NK cells, T cells, γ-δ T cells, and/or NKT cells, is 100 or more times the average value of the typical number of each blood cell per unit volume in comparison using the same liquid volume as in the amount of blood used in culture.

### 5-3. Method

Hereinafter, a method for administering the NK cell-enriched blood product in the cellular immunotherapy of this disclosure will be described by taking adoptive immunotherapy as an example. The administration method is basically the same as a known method performed in the conventional adoptive immunotherapy except that the NK cell-enriched blood product of the first embodiment is administered. Thus, the administration method can be performed according to that of the adoptive immunotherapy known in the art. Examples thereof include methods involving administering the blood product produced by the method for producing an NK cell-enriched blood product of the first embodiment from blood collected from a patient, into the body of the patient using, for example, intravenous injection or drip infusion approximately 2 weeks later.

One dose of the NK cell-enriched blood product according to this embodiment can be a volume containing NK cells in the range of 5 × 10⁷ to 4 × 10⁹ cells for a human. This dose is intended for a general adult. For actual administration, it is preferred to appropriately adjust the dose in consideration of the age, sex, body weight, disease conditions, body strength, etc., of a recipient of the blood product.

One example of the cellular immunotherapy of this disclosure includes 1 course (6 cycles) or longer of continuous administration at approximately 2-week intervals with the above-described administration method defined as one cycle. Cellular immunotherapy other than adoptive immunotherapy can also be performed in the same way as above except that an NK cell-enriched blood product obtained from a non-self organism is administered.

### 5-4. Effect

The cellular immunotherapy of this disclosure has high efficacy on the healing of a disease such as cancer, compared with many conventional immunotherapies, particularly, adoptive immunotherapy. A person skilled in the conventional adoptive immunotherapy can carry out the cellular immunotherapy of this embodiment without acquiring particular skills because this cellular immunotherapy can be operated by the same basic technique, etc., as in the conventional adoptive immunotherapy.

### Examples

Hereinafter, the present invention will be described specifically with reference to Examples. Examples below are provided merely for illustrative purposes, and the present invention is not intended to be limited to these Examples by any means. In this context, small experimental errors and deviations are tolerated for numeric values as to temperature, amount, time, etc., used in these Examples.

### [Example 1]

### <Method for producing NK cell-enriched blood product using blood of healthy donor>

The first embodiment of the present invention will be described with reference to a specific example of a method for producing the blood product used in adoptive immunotherapy.

### (1) Preparation of autologous plasma

First, autologous plasma for culture was prepared from donor organisms. The donors were healthy individuals (four males in their 30s to 40s; designated as donors #1, 2, 3, and 4). 50 mL of peripheral whole blood was collected from the vein of each donor into a blood collection tube supplemented with 50 units/mL heparin. The collected peripheral whole blood was transferred to a sterile conical centrifuge tube and centrifuged at 3000 rpm for 10 minutes. Then, the supernatant was separated as plasma. To the remaining blood cell components separated from plasma, sterile PBS (-) or a medium for culture was added in an amount 3 times that of the whole blood before plasma separation to prepare a blood cell component solution, which was in turn used in the subsequent preparation of peripheral blood mononuclear cells. The plasma was inactivated by treatment at 56°C for 30 minutes and further centrifuged at 3000 rpm for 10 minutes to remove platelet, etc. Then, the plasma was stored at 4°C. This plasma was intended for addition to a cell culture medium and used in a necessary amount every time a medium was prepared.

### (2) Preparation of peripheral blood mononuclear cells (PBMCs)

A specific gravity solution was layered onto the blood cell component solution. Erythrocytes or granulocytes were removed using a density-gradient centrifugation method to isolate PBMCs. The specific gravity solution used was Ficoll-Paque PLUS (Amersham Biosciences), and the operational procedures followed the protocol supplied with the kit. The collected PBMCs were washed 2 or 3 times by the addition of 30 mL of serum-free PBS (-) or medium for cultured cells. The medium for cultured cells used was, for example, a serum-free RPMI1640 medium. After the washing, an aliquot was sampled from the obtained suspension of PBMCs and stained with a Turk's solution, and the number thereof was then counted using a hemacytometer. As a result, 4 × 10⁷ PBMCs were collected from 50 mL of peripheral whole blood.

The peripheral blood mononuclear cells thus collected were added and suspended at a cell density of 1 × 10⁶ cells/mL to OpTmizer (Life Technologies Corp.) medium supplemented with 5% (V/V) of the autologous plasma.

### (3) Stimulation step

0.2 mg of an anti-CD16 antibody (Clone 3 GB, Beckman Coulter, Inc. Coulter, Inc.) was dissolved in 1 mL of sterile distilled water. Since this anti-CD16 antibody is not a sterile product, this solution was sterilized by filtration through a 0.22 µm filter. The solution was adjusted to 1 µg/mL in terms of the final concentration with 199 mL of sterile distilled water, and mixed. After filtration, 5 mL of the anti-CD16 antibody solution was placed in a 25 cm² culture flask and left standing overnight at 37°C to immobilize the anti-CD16 antibody in this solution onto the inner wall of the flask. Then, the solution was discarded, and the inside of the flask was washed twice with sterile PBS (-).

Five milliliter of the prepared suspension of PBMCs was transferred into the flask. Subsequently, 8.5 µL of a 1.7 µL/mL aqueous zoledronic acid (Zometa; Novartis Pharma K.K.) solution, 20 µL of a 4 µL/mL aqueous OK432 (Picibanil; Chugai Pharmaceutical Co., Ltd.) solution, and 4 µL of 900 units/µL IL-2 (Proleukin; Chiron Corp.) solution were added to the suspension of PBMCs, and the mixture was sufficiently stirred.

In order to sufficiently stimulate PBMCs with each of the NK cell growth-stimulating factors, the medium was transferred to a 5% CO₂ incubator preset to a chamber temperature of 37°C, and kept for 3 days.

### (4) Culture step

In order to remove the NK cell growth-stimulating factors from the culture solution, 5 mL of the culture solution was then collected into a conical centrifuge tube and centrifuged at 1200 rpm for 8 minutes. After the centrifugation, the medium in the supernatant was removed, and the cell pellet was suspended in 4 mL of OpTmizer medium containing 5% (V/V) autologous serum containing 700 units/µL IL-2. The collected cell suspension was transferred to a new, anti-CD16 antibody-unimmobilized flask and cultured again for 13 days in a 5% CO₂ incubator set to 37°C. The OpTmizer medium containing 5% (V/V) autologous serum was replaced by a fresh one every 2 to 4 days. In this way, the NK cell-enriched blood product of the second embodiment of the present invention was prepared.

For actually using the NK cell-enriched blood product, it is required to perform a contamination test or pretreatment. Hereinafter, their procedures will be described simply.

### (Contamination test)

The presence or absence of endotoxin in the culture solution was confirmed using Limulus ES-II (Wako Pure Chemical Industries, Ltd.) according to the protocol supplied with the kit. Also, the presence or absence of bacteria or fungus was confirmed by colony formation assay using an aliquot of the culture solution applied to an agar medium.

### (Pretreatment of NK cell-enriched blood product)

Two weeks after culture, the culture solution was transferred to a centrifuge tube and centrifuged at 1200 rpm for 10 minutes, and the supernatant was then discarded. The precipitates were suspended by the addition of 50 mL of PBS (-) and centrifuged again at 1200 rpm for 10 minutes, and the supernatant was then discarded. This operation was performed 3 repetitive times to remove the medium components. Finally, the residue was suspended in 70 mL of the lactate Ringer solution. In this way, the NK cell-enriched blood product was obtained as the final product.

### [Example 2]

### <Growth of NK cells - (1) ->

In order to confirm that the method for producing an NK cell-enriched blood product of the present invention did not require an activation step and was more advantageous in production process than the invention according to the prior application, the growth rate of NK cells was examined using blood derived from the healthy individual (donor #1) described in Example 1.

### (Method)

In this Example, three samples shown below were examined for the growth rate of NK cells using zoledronic acid hydrate (Zometa; Novartis Pharma K.K.) as a bisphosphonate derivative or the like and IL-2 as a cytokine.

Sample A: The NK cell growth-stimulating factors used were 1 µg/mL anti-CD16 antibody, 0.01 KE/mL OK432, and 700 units/mL IL-2 (concentrations were all indicated by the final concentrations). The NK cell growth-stimulating factors in this sample correspond to growth-stimulating factors used in the prior application except that, unlike the production method according to the prior application, the sample of this Example did not undergo the activation step of keeping collected blood at 38°C to 40°C for 10 hours to 30 hours.

Sample B: The NK cell growth-stimulating factors used were 1 µg/mL anti-CD16 antibody, 0.01 KE/mL OK432, 5 µM/mL zoledronic acid, and 700 units/mL IL-2 (concentrations were all indicated by the final concentrations). The NK cell growth-stimulating factors in this sample correspond to the NK cell growth-stimulating factors of the present invention.

Sample C: 5 µM/mL zoledronic acid and 700 units/mL IL-2 were used as a control for sample (B).

The basic production method followed Example 1. As described above, for sample A, no zoledronic acid was added, and the essential step of treating blood at 38°C to 40°C for 10 hours to 30 hours (activation step) in the method of the prior application was not performed. For sample C, neither an anti-CD16 antibody nor OK432 was added. The day when PBMCs were suspended at a cell density of 1 × 10⁶ cells/mL in OpTmizer medium was defined as day 0. The day when stimulation and culture were initiated by stimulation with each stimulating factor and addition of 10% autologous plasma to the medium was defined as day 1. At culture (including the stimulation step) days 0, 3, 5, 7, 9, 11, and 13, an aliquot of each culture solution was collected, and the number of cultured cells in each culture solution was determined.

NK cells in the cultured cells were assayed using a flow cytometry analysis method. Specifically, the NK cells in the blood product were immunostained using a combination of fluorescent material-labeled monoclonal antibodies (ECD-labeled anti-CD3 antibody and PC5-labeled anti-CD56 antibody; Immunotech). The immunostaining was performed by adding, to the cell suspension, each antibody in an amount recommended by the document supplied with the antibody, and staining the cells at room temperature for 15 minutes in the dark, followed by centrifugation and washing off of the supernatant containing the fluorescently labeled antibodies. Subsequently, the kinetics of the NK cells were assayed by flow cytometry using Cytomics FC500 (Beckman Coulter, Inc.) based on the combination of the antibodies. The assay data was analyzed by CXP analysis.

### (Results)

The results are shown in Figures 1 and 2 and Table 1. Figure 1 shows a cytogram at culture day 11 as one example of the respective flow cytometry analysis results of the samples A to C. In Figure 1, the abscissa and the ordinate represent the fluorescence intensities of the ECD-labeled anti-CD3 antibody and the PC5-labeled anti-CD56 antibody, respectively, on a log scale. Four zones (X1 to X4) in each cytogram were divided as X1 (CD3⁻CD56⁺): a fraction of NK cells, X2 (CD3⁺CD56⁺) and X4 (CD3⁺CD56⁻): fractions of CD3-positive T lymphocytes, and X3 (CD3⁻CD56⁻): a fraction of the other cells, such as B cells, based on these two types of fluorescence intensities. The numeric value in each fraction represents the ratio (%) of the cells contained in the fraction to all the assayed cultured cells. Figure 2 shows the relationship between lapse time in the culture period and the number of cells in the culture solution for each of the samples A to C. Table 1 shows the total number of cells at culture days 0 and 13 (the total number of cells at day 0 corresponds to the total number of PBMCs), the ratio (NK%) of NK cells to the total number of cells, the growth rate of NK cells, etc., for the samples A and B.

All of these samples hardly differed until day 5 or so. The sample A was supplemented with the same NK cell growth-stimulating factors as in the prior application, but did not undergo the activation step of treating blood at a particular temperature for a particular time. Thus, the subsequent growth of NK cells was also low. By contrast, the sample B, which was cultured under the same conditions thereas except for NK cell growth-stimulating factors, exhibited significant increase in the number of NK cells from days 9 to 13. At day 13, the growth rate of NK cells reached approximately 9 times that of the sample A (see Table 1). These results demonstrated that the production method of the present invention was able to efficiently grow NK cells without the need of the activation step. As is further evident from the sample C, the addition of only zoledronic acid and IL-2 was insufficient for increasing the number of cells. This result demonstrated that all of the anti-CD16 antibody, OK432, zoledronic acid, and IL-2 were necessary for the efficient growth of NK cells in the production method of the present invention.

**[Table 1]**

| At start of culture (day 0) | | | | | |
|---|---|---|---|---|---|
| Donor (healthy individual) | Sample | Total number of cells (x10⁶) | NK % | | |
| #1 | A | 5 | 19.3 | | |
| | B | 5 | 19.3 | | |

| Culture day 13 | | | | | |
|---|---|---|---|---|---|
| Donor (healthy individual) | Sample | Total number of cells (x10⁶) | NK % | NK growth rate (-fold) | Cytotoxic activity of NK (E/T=3:1) |
| #1 | A | 79.0 | 84.4 | 68.7 | 69.9 |
| | B | 741.6 | 80.3 | 614.0 | 68.1 |

### [Example 3]

### <Growth of NK cells - (2) ->

In order to confirm the effect of zoledronic acid on the method for producing an NK cell-enriched blood product according to the prior application, the growth rate of NK cells was examined using blood derived from the healthy individuals (donors #2 and 3) described in Example 1.

### (Method)

Two samples shown below were examined for the growth rate of NK cells.

Sample D: The NK cell growth-stimulating factors used were 1 µg/mL anti-CD16 antibody, 0.01 KE/mL OK432, and 700 units/mL IL-2 (concentrations were all indicated by the final concentrations).

Sample E: The NK cell growth-stimulating factors used were 1 µg/mL anti-CD16 antibody, 0.01 KE/mL OK432, 5 µM/mL zoledronic acid, and 700 units/mL IL-2 (concentrations were all indicated by the final concentrations).

As described above, the NK cell growth-stimulating factors added to samples D and E in this Example were the same as the samples A and B, respectively, in Example 2. This Example differed from Example 2 in that this Example followed the method for producing an NK cell-enriched blood product according to the prior application as a rule. Specifically, after a growth-stimulating factor addition step (which corresponds to the stimulation step in the present invention), the medium was kept overnight at 39°C for high-temperature stimulation.

The number of NK cells was determined using a flow cytometry analysis method as in Example 2.

### (Results)

The results are shown in Table 2. This table shows the total number of cells at culture days 0 and 13, the ratio (NK%) of NK cells to the total number of cells, the growth rate of NK cells, etc., for the samples D and E of each donor. In the sample D, the growth rate of NK cells from the number of NK cells before culture reached 245 times (#2) and 160 times (#3) at day 13. By contrast, in the sample E supplemented with zoledronic acid, the growth rate of NK cells from the number of NK cells before culture reached approximately 325 times (#2) and 290 times (#3) at day 13. This result demonstrated that the method for producing an NK cell-enriched blood product of the present invention involving the addition of zoledronic acid had approximately 1.3 to 1.8 times higher growth rate of NK cells even under the same conditions as in the production method according to the prior application. This means that the production method of the present invention further combined with the activation step can produce a much higher growth rate of NK cells than that of the production method according to the prior application.

**[Table 2]**

| At start of culture (day 0) | | | | | |
|---|---|---|---|---|---|
| Donor (healthy individual) | Sample | Total number of cells (x10⁶) | NK % | | |
| #2 | D | 9 | 11 | | |
| | E | 9 | 11 | | |
| #3 | D | 7.5 | 12.3 | | |
| | E | 7.5 | 12.3 | | |
| | | | | | |

| Culture day 13 | | | | | |
|---|---|---|---|---|---|
| Donor (healthy individual) | Sample | Total number of cells (x10⁶) | NK % | NK growth rate (-fold) | Cytotoxic activity of NK (E/T=6:1) |
| #2 | D | 283.0 | 85.7 | 245.0 | 84.0 |
| | E | 446.0 | 72.3 | 325.8 | 85.0 |
| #3 | D | 343.2 | 42.8 | 159.7 | 83.0 |
| | E | 478.0 | 56.2 | 292.0 | 84.0 |

### [Example 4]

### <Growth of NK cells in NK cell-enriched blood product derived from frozen blood>

An NK cell-enriched blood product was produced using frozen blood derived from the healthy donor #4 and examined for the effect of the production method of the present invention on the growth of NK cells in frozen blood.

### (Method)

The procedures of the method for producing an NK cell-enriched blood product were basically the same as in Example 1 except that blood was collected from the donor #4 and then, this blood or, for example, PBMCs isolated by appropriate treatment were temporarily frozen and then used in the production method. Thus, the procedures different from Example 1 will be described below specifically, and the description about the other (same) procedures is omitted.

First, 50 mL of peripheral whole blood was collected from the vein of the donor #4 into a blood collection tube supplemented with 50 units/mL heparin. The collected peripheral whole blood was transferred to a sterile conical centrifuge tube and centrifuged at 3000 rpm for 10 minutes. Then, the supernatant was separated as plasma. PBMCs were prepared in the same way as in Example 1 from the remaining blood cell components separated from plasma. The PBMCs were directly frozen, or after addition of 1 mL of a freezing agent (Bambanker; Nippon Genetics Co., Ltd.), placed in a freezing tube and stored in a deep freezer of -80°C.

The thawing operation was performed by rapidly thawing the frozen cells, centrifuging them, washing off the solution for freezing, and adding thereto a culture solution. Subsequently, the samples A to C were handled, as a rule, in the same way as the procedures subsequent to "(3) Stimulation step" described in Example 1.

### (Results)

The results are shown in Figure 3 and Table 3.

Figure 3 shows a cytogram at culture day 11 as one example of the respective flow cytometry analysis results of the samples A, B, and C, as in Example 2. Four zones (X1 to X4) in each cytogram are the same as in Example 2. Table 3 shows the absolute number of NK cells at culture days 0 and 12, growth rate, etc., for the samples A and B of the blood donor #4. For all of the samples A to B, the pattern of cells distributed in each zone of Figure 3 was substantially the same as that of cell distribution shown in Figure 1.

Table 3 shows the absolute number of NK cells at culture days 0 and 12, growth rate, etc., for the samples A and B of the blood donor #4. In the sample A, the growth rate of NK cells from culture day 0 reached 150 times at day 12. By contrast, in the sample B supplemented with zoledronic acid, the growth rate of NK cells from culture day 0 reached approximately 380 times at day 12. These results demonstrated that the production method of the present invention even using frozen blood can produce a higher growth rate of NK cells than that of the production method according to the prior application, as in use of fresh blood.

**[Table 3]**

| At start of culture (day 0) | | | | |
|---|---|---|---|---|
| Donor (healthy individual) | Sample | Total number of cells (x10⁶) | NK% | |
| #4 | A | 5 | 12.1 | |
| | B | 5 | 12.1 | |
| | | | | |

| Culture day 12 | | | | |
|---|---|---|---|---|
| Donor (healthy individual) | Sample | Total number of cells (x10⁶) | NK% | NK growth rate (-fold) |
| #4 | A | 176.4 | 51.0 | 150.0 |
| | B | 485.8 | 46.6 | 377.3 |

### [Example 5]

### <Assay of activated NK cells derived from healthy donor blood: Cytotoxic activity against K562>

The activation of NK cells in the NK cell-enriched blood product of the present invention was assayed on the basis of cytotoxic activity against a K562 cell line, which was targeted by NK cells.

### (Method)

First, established leukemia cell line K562 cells were labeled with a fluorescent dye Calcein-AM. The labeling was performed by incubation at 37°C for 30 minutes in a RPMI-1640 medium (containing 10% fetal bovine serum) supplemented with a 1/100 volume of Calcein-AM solution (Dojindo Laboratories). The cells thus labeled were washed with PBS (-) and used as target K562 cells. Next, NK cells in the sample A- and sample B-derived NK cell-enriched blood products (which correspond to Figures 4A and 4B, respectively, described later) produced by the method of Example 2, an NK cell line KHYG-1, and NK cells in the sample D- and sample E-derived NK cell-enriched blood products produced by the method of Example 3 were separately used as effector cells (E). These effector cells were adjusted to their respective predetermined values in terms of the ratio (E/T ratio) to the target K562 cells (target cells: T), then separately placed in a 96-well plate, and reacted at 37°C for 2 hours at a CO₂ concentration of 5%. After the reaction, the amounts of the target cells that retained fluorescence, i.e., survived, were detected on the basis of their fluorescence intensities using Terascan VP (Minerva Tech K.K.). The value of cytotoxic activity against K562 was calculated by comparison with a control before cytotoxicity, i.e., fluorescence intensity from a state unsupplemented with effector cells.

### (Results)

The cytotoxic activity of the NK cells produced by the method of Example 2 from the healthy donor #1 is shown in Table 1. The cytotoxic activity of the NK cells produced by the method of Example 3 from the healthy donors #2 and 3 is shown in Table 2. The cytotoxic activity of the NK cells produced by the method of Example 4 from the healthy donor #4 is shown in Figure 5. The E/T ratios used are shown in the corresponding tables or diagram.

As is evident from Tables 1 and 2 and Figure 5, the NK cells obtained in the method for producing an NK cell-enriched blood product of the present invention had cytotoxic activity substantially equivalent to that of the method for producing an NK cell-enriched blood product according to the prior application. This result demonstrated that the method for producing an NK cell-enriched blood product of the present invention was able to provide the cytotoxic activity of NK cells as high as that obtained in the production method according to the prior application, and to further enhance the cytotoxic activity of the NK cells by increasing the number of the NK cells.

### [Example 6]

### <Growth of γ-δ T cells>

The zoledronic acid used in the method for producing an NK cell-enriched blood product of the present invention is known to enhance the growth activity of γ-δ T cells (Sato K, et al., 2005, Int. J. Cancer, 116: 94-99; and Kondo M, et al., 2008, Cytotherapy, 10 (8): 842-856.). Thus, the production method of the present invention was also examined for whether or not it was able to grow γ-δ T cells while improving the growth activity of NK cells.

### (Method)

The blood used was each of fresh blood collected from the healthy donors #2 and 3 and frozen PBMCs obtained by treating the fresh blood and temporarily cryopreserving the cells. Since these blood samples (PBMCs) also contained the γ-δ T cells of interest, the basic operational procedures were performed according to Examples 3 and 4 above.

The number γ-δ T cells was determined using a flow cytometry analysis method, as in the NK cells. The γ-δ T cells in the blood product were immunostained using a combination of fluorescent material-labeled monoclonal antibodies (PC5-labeled anti-PNA γ-δ T antibody and ECD-labeled anti-CD3 antibody; Immunotech). The immunostaining was performed by adding, to the cell suspension, each antibody in an amount recommended by the document supplied with the antibody, and staining the cells at room temperature for 15 minutes in the dark, followed by centrifugation and washing off of the supernatant containing the fluorescently labeled antibodies. Subsequently, the kinetics of the γ-δ T cells were assayed by flow cytometry using Cytomic FC500 (Beckman Coulter, Inc.) based on the combination of the antibodies. The assay data was analyzed by CXP analysis.

### (Results)

The results are shown in Figures 6 and 7 and Table 4.

Figures 6 and 7 show a cytogram (only at culture day 11 as one example) of the respective flow cytometry analysis results of the samples A, B, and C (which corresponds to A, B, and C, respectively, in Figures 6 and 7) of the healthy donor #2 using fresh blood and frozen PMBCs, respectively. In Figures 6 and 7, the abscissa and the ordinate represent the fluorescence intensities of the ECD-labeled anti-CD3 antibody and the PC5-labeled anti-PAN γ-δ T antibody, respectively, on a log scale. Four zones (Y1 to Y4) in each cytogram were divided as Y1 (CD3⁻PAN⁺): an unstained (qualified cells were absent in this staining) fraction, Y2 (CD3⁺PAN⁺): a fraction of γ-δ T cells, Y4 (CD3⁺PAN⁻): a fraction of α-β T cells, and Y3 (CD3⁻PAN⁻): a fraction of cells (including NK cells) other than T cells based on these two types of fluorescence intensities. The numeric value in each fraction represents the ratio (%) of the cells contained in the fraction to all the assayed cultured cells.

Table 4 shows the total number of cells at culture day 0, the ratio (γδT%) of γ-δ T cells thereto, the total number of cells at culture day 13, the ratio (γδT%) of γ-δ T cells thereto, growth rate, etc., for the samples A and B from the fresh blood of the healthy donors #2 and #3.

As is evident from Figures 6 and 7, cell% in the fraction Y2 of γ-δ T cells was as high as approximately 40% for the sample C supplemented with zoledronic acid and IL-2, but was only a small percent for the sample A unsupplemented with zoledronic acid. By contrast, for the sample B corresponding to the production method of the present invention, cell% in the fraction Y2 was 12% and 19%, which were both higher compared with the sample A. This is also evident from the growth rate of γ-δ T cells in the samples A and B of Table 4. These results demonstrated that the method for producing an NK cell-enriched blood product of the present invention was able to enhance the growth of NK cells without impairing the γ-δ T cell growth-enhancing effect of zoledronic acid.

The γ-δ T cells, as in NK cells, can kill target cells without targeting MHC class I and as such, can be used in cellular immunotherapy. Hence, the production method of the present invention can advantageously perform the simultaneous growth of NK cells and γ-δ T cells, which has not been achieved by the method for producing an NK cell-enriched blood product according to the prior application.

**[Table 4]**

| At start of culture (day 0) | | | | |
|---|---|---|---|---|
| Donor (healthy individual) | Sample | Total number of cells (x10⁶) | γ-δT % | |
| #2 | A | 9 | 7.5 | |
| | B | 9 | 7.5 | |
| #3 | A | 7.5 | 8.3 | |
| | B | 7.5 | 8.3 | |
| | | | | |

| Culture day 13 | | | | |
|---|---|---|---|---|
| Donor (healthy individual) | Sample | Total number of cells (x10⁶) | γ-δT % | γ-δ T growth rate (-fold) |
| #2 | A | 283.0 | 2.6 | 10.9 |
| | B | 446.0 | 20.0 | 131.2 |
| #3 | A | 343.2 | 1.6 | 8.9 |
| | B | 478.0 | 6.4 | 49.4 |

### [Example 7]

### <Method for producing NK cell-enriched blood product using blood of cancer patient donor and various examinations>

In Examples 1 to 6 above, the NK cell-enriched blood product was produced by the method of the present invention using healthy individual-derived blood and examined for the growth rate of NK cells, the cytotoxic activity of NK cells, the growth rate of γ-δ T cells, etc., brought about by this method. As a result, the higher growth rate and cytotoxic activity of NK cells were obtained, compared with conventional methods or the production method according to the prior application.

However, it was uncertain whether these effects obtained using healthy individual-derived blood were also produced by the method for producing an NK cell-enriched blood product of the present invention using cancer patient-derived blood.

Thus, informed consent was obtained from 10 cancer patients having various cancers at various stages, and an NK cell-enriched blood product was produced in the same way as in Examples above using blood derived from these cancer patients and examined for the growth rate of NK cells, the cytotoxic activity of NK cells, the growth rate of γ-δ T cells, etc.

### (1) Method for producing NK cell-enriched blood product

The blood donors who provided autologous plasma for culture were 10 individuals (donors #1p to 10p) described in Table 5. As shown in Table 5, these cancer patients differed in terms of age, sex, primary cancer type, and cancer stage. The production method was performed by procedures similar to the method described in Example 1.

**[Table 5]**

| Donor (cancer patient) | Age | Sex | Stage | Disease name |
|---|---|---|---|---|
| #1p | 66 | Male | IV | Gastric cancer metastasized to liver |
| #2p | 77 | Female | IV | Gastric cancer metastasized to liver |
| #3p | 79 | Male | IV | Prostatic cancer multiply metastasized to bone |
| #4p | 82 | Female | IV | Colon cancer (recurrence) metastasized to liver and lung |
| #5p | 73 | Male | IV | Lung cancer |
| #6p | 47 | Female | Ia | Lung cancer |
| #7p | 44 | Female | IIIb | Breast cancer |
| #8p | 7 | Female | - | Malignant glioma |
| #9p | 86 | Male | IV | Lung cancer metastasized bone |
| #10p | 67 | Male | IIIb | Lung cancer |

### (2) Growth of NK cells

In order to confirm that the effect of zoledronic acid was obtained even using cancer patient-derived blood, the growth rate of NK cells was examined using the blood of all the cancer patients described in the preceding paragraph (1) of this Example.

### (Method)

The basic procedures followed the method described in Example 3 except that: in this Example, the NK cell growth-stimulating factors used were the same as in the sample E described in Example 3; high-temperature stimulation was performed; and the culture period was 12, 17, 19, or 21 days for each patient. After the completion of culture, an aliquot of each culture solution was collected, and the number of NK cells in each culture solution was determined.

### (Results)

The results are shown in Table 6.

**[Table 6]**

| At start of culture (day 0) | | | | | |
|---|---|---|---|---|---|
| Donor (cancer patient) | Total number of cells (x10⁶) | NK % | | | |
| Average (n=10) | 26.4 | 15.1 | | | |
| Standard deviation | 8.7 | 5.5 | | | |
| | | | | | |

| At completion of culture | | | | | |
|---|---|---|---|---|---|
| Donor (cancer patient) | The number of culture days | Total number of cells (x10⁶) | NK % | NK growth rate (-fold) | Cytotoxic activity of NK % (E/T;6/1) |
| Average (n=10) | 17.2 | 2131.7 | 50.5 | 448.3 | 84.6% |
| Standard deviation | 3.0 | 1194.7 | 20.0 | 492.5 | 5.7% |

Table 6 shows the total number of cells at culture day 0 and after the completion of culture for each patient, the number of culture days, the ratio (NK%) of NK cells to the total number of cells, and the growth rate of NK cells, etc., indicated by the average of 10 patients and standard deviation. The average growth rate of NK cells from the number of NK cells before culture was as high as approximately 450 times, though the number of culture days varied widely from 12 to 21 days among patients.

### (3) Assay of activated NK cells: Cytotoxic activity against K562

The activation of NK cells produced from cancer patient-derived blood was assayed on the basis of cytotoxic activity against a K562 cell line, which was targeted by NK cells.

### (Method)

The basic procedures followed the method described in Example 5. The effector cells used were NK cells in the NK cell-enriched blood product that was derived from the blood of each of 10 cancer patient and produced in the preceding paragraph (2) of this embodiment.

### (Results)

The results are shown in Table 6. According to the method for producing an NK cell-enriched blood product of the present invention, even NK cells derived from cancer patient blood exhibited cytotoxic activity as very high as 84.6% (standard deviation: 5.7%) on average. This result demonstrated that the method of the present invention was able to enhance the growth activity of NK cells.

### (4) Growth of γ-δ T cells

The method for producing an NK cell-enriched blood product of the present invention was examined for whether it was able to grow γ-δ T cells in a product even using cancer patient-derived blood, as in use of healthy individual-derived blood.

### (Method)

The blood used was only fresh blood collected from 10 cancer patients shown in Table 5. The basic procedures followed Example 6 above except that: the NK cell growth-stimulating factors used were the same as in the sample E; high-temperature stimulation was performed; and the culture period was 12, 17, 19, or 21 days for each patient.

### (Results)

The results are shown in Table 7.

**[Table 7]**

| At start of culture (day 0) | | | | |
|---|---|---|---|---|
| Donor (cancer patient) | Total number of cells (x10⁶) | γ-δT% | | |
| Average (n=10) | 26.4 | 1.2 | | |
| Standard deviation | 8.7 | 1.0 | | |

| At completion of culture | | | | |
|---|---|---|---|---|
| Donor (cancer patient) | The number of culture days | Total number of cells (x10⁶) | γ-δT% | γ-δ T growth rate (-fold) |
| Average (n=10) | 17.2 | 2131.7 | 23.7 | 2953.6 |
| Standard deviation | 3.0 | 1194.7 | 17.3 | 2404.0 |

Table 7 shows the total number of cells at culture day 0 and after the completion of culture for each cancer patient, the number of culture days, the ratio (γδT %) of γ-δ T cells to the total number of cells, and the growth rate of γ-δ T cells, etc., indicated by the average of 10 patients and standard deviation. The results of Table 7 demonstrated that according to the method for producing an NK cell-enriched blood product of the present invention, even the NK cell-enriched blood product derived from cancer patient-derived blood had γ-δ T cell growth-enhancing effect.

The results of this Example demonstrated that according to the method for producing an NK cell-enriched blood product and the composition for NK cell enrichment of the present invention even using cancer patient-derived blood can produce the same effects as in use of healthy individual-derived blood, irrespective of the age, sex, cancer type, and cancer stage of donors. As a result, the method for producing an NK cell-enriched blood product of the present invention can employ blood collected from cancer patients themselves. Thus, a composition for NK cell enrichment produced therefrom can be brought back into patient's body such that NK cells or γ-δ T cells having potentiated cytotoxic activity can be introduced in large amounts into the body without rejection by virtue of autologous cells. Thus, the method of the present invention achieves adoptive immunotherapy exceedingly desirable for cancer treatment.

## Claims

1. A method for producing an NK cell-enriched blood product, comprising:
a stimulation step of stimulating NK cells comprised in blood collected from an organism, with NK cell growth-stimulating factors comprising at least an anti-CD16 antibody, OK432 (Picibanil), a bisphosphonate derivative or a salt thereof, or a hydrate thereof, and a cytokine; and
a culture step of culturing the blood at a physiological cell temperature after the stimulation step,
wherein the bisphosphonate derivative is a compound represented by the following general formula 1: 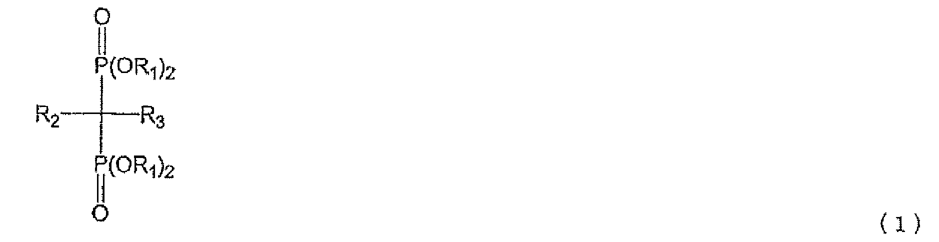
in the formula, R₁ represents a hydrogen atom (H) or a lower alkyl group; R₂ and R₃ each independently represent a hydrogen atom, halogen, a hydroxyl group, an amino group, a thiol group, a substituted or unsubstituted aryl group, a substituted or unsubstituted alkyl group, a lower alkylamino group, an aralkyl group, a cycloalkyl group, or a heterocyclic group, or R₂ and R₃ form a portion of a cyclic structure containing them wherein substituents forming the cyclic structure are each independently derived from halogen, a lower alkyl group, a hydroxyl group, a thiol group, an amino group, an alkoxy group, an aryl group, an arylthio group, an aryloxy group, an alkylthio group, a cycloalkyl group, or a heterocyclic group in R₂ and R₃.

2. The method according to claim 1, wherein the stimulation step comprises keeping the NK cells at 38°C to 40°C for 10 hours to 30 hours to apply thereto high-temperature stimulation.

3. The method according to claim 1 or 2, wherein the bisphosphonate derivative is selected from the group consisting of zoledronic acid, pamidronic acid, alendronic acid, risedronic acid, ibandronic acid, incadronic acid, etidronic acid, and a combination thereof.

4. The method according to any one of claims 1 to 3, wherein the cytokine is IL-2.

5. The method according to any one of claims 1 to 4, wherein the anti-CD 16 antibody is immobilized on a solid-phase support.

6. The method according to any one of claims 1 to 5, wherein the culture period in the culture step is 9 days to 21 days.

7. The method according to any one of claims I to 6, wherein the blood is peripheral blood, cord blood, or bone marrow fluid.

8. A composition for NK cell enrichment comprising an anti-CD16 antibody, OK432, a bisphosphonate derivative or a salt thereof, or a hydrate thereof, and a cytokine,
wherein the bisphosphonate derivative is a compound represented by the following general formula 1: 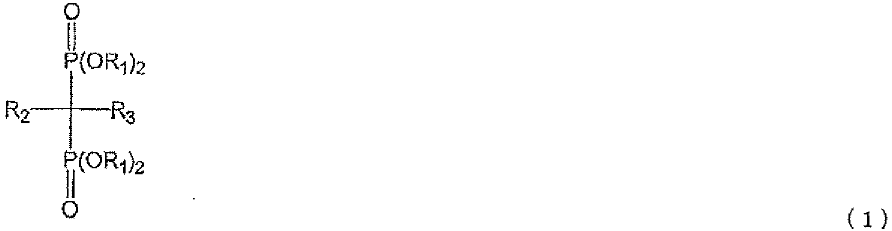 in the formula, R₁ represents a hydrogen atom (H) or a lower alkyl group; R₂ and R₃ each independently represent a hydrogen atom, halogen, a hydroxyl group, an amino group, a thiol group, a substituted or unsubstituted aryl group, a substituted or unsubstituted alkyl group, a lower alkylamino group, an aralkyl group, a cycloalkyl group, or a heterocyclic group, or R₂ and R₃ form a portion of a cyclic structure containing them wherein substituents forming the cyclic structure are each independently derived from halogen, a lower alkyl group, a hydroxyl group, a thiol group, an amino group, an alkoxy group, an aryl group, an arylthio group, an aryloxy group, an alkylthio group, a cycloalkyl group, or a heterocyclic group in R₂ and R₃.

9. The composition according to claim 8, wherein the bisphosphonate derivative is selected from the group consisting of zoledronic acid, pamidronic acid, alendronic acid, risedronic acid, ibandronic acid, incadronic acid, etidronic acid, and a combination thereof.

10. The composition according to claim 8 or 9, wherein the cytokine is IL-2.

11. A kit for production of NK cell-enriched blood comprising an anti-CD 16 antibody, OK432, a bisphosphonate derivative or a salt thereof, or a hydrate thereof, and a cytokine,
wherein the bisphosphonate derivative is a compound represented by the following general formula 1: 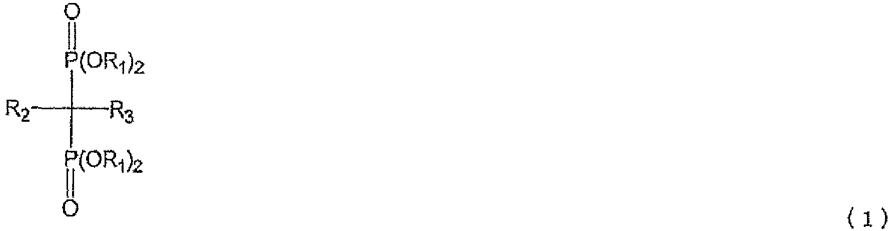 in the formula, R₁ represents a hydrogen atom (H) or a lower alkyl group; R₂ and R₃ each independently represent a hydrogen atom, halogen, a hydroxyl group, an amino group, a thiol group, a substituted or unsubstituted aryl group, a substituted or unsubstituted alkyl group, a lower alkylamino group, an aralkyl group, a cycloalkyl group, or a heterocyclic group, or R₂ and R₃ form a portion of a cyclic structure containing them wherein substituents forming the cyclic structure are each independently derived from halogen, a lower alkyl group, a hydroxyl group, a thiol group, an amino group, an alkoxy group, an aryl group, an arylthio group, an aryloxy group, an alkylthio group, a cycloalkyl group, or a heterocyclic group in R₂ and R₃.

12. The kit according to claim 11, wherein the bisphosphonate derivative is selected from the group consisting of zoledronic acid, pamidronic acid, alendronic acid, risedronic acid, ibandronic acid, incadronic acid, etidronic acid, and a combination thereof.

13. The kit according to claim 11 or 12, wherein the cytokine is IL-2.

## Patentansprüche

1. Verfahren zur Produktion eines mit NK-Zellen angereicherten Blut-Produktes, umfassend:
einen Stimulationsschritt zur Stimulierung von NK-Zellen, die im Blut, gesammelt aus einem Organismus, umfasst sind, mit NK-Zell wachstumsstimulierenden Faktoren, die mindestens einen anti-CD16 Antikörper, OK432 (Picibanil), ein Bisphosphonat-Derivat oder Salz davon, oder ein Hydrat davon, und ein Zytokin umfassen; und
einen Kulturschritt zur Kultivierung von Blut bei einer physiologischen Zelltemperatur nach dem Stimulationsschritt,
wobei das Bisphosphonat-Derivat ein Stoff ist mit folgender Formel (1): 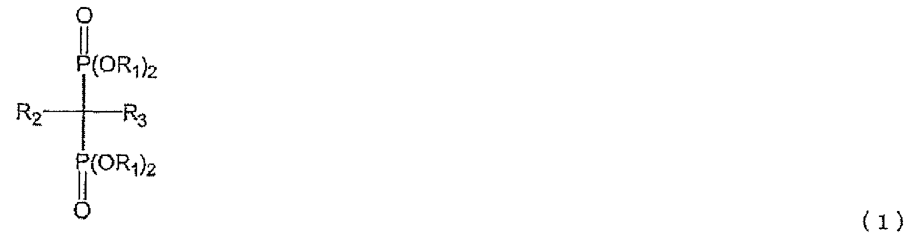
in der Formel, R₁ steht für ein Wasserstoffatom (H) oder eine niedere Alkyl Gruppe; R₂ und R₃ repräsentieren unabhängig voneinander ein Wasserstoffatom, Halogen, eine Hydroxylgruppe, eine Aminogruppe, eine Thiolgruppe, eine substituierte oder unsubstituierte Arylgruppe, eine substituierte oder unsubstituierte Alkylgruppe, eine niedere Alkylaminogruppe, eine Aralkylgruppe, eine Zykloalkylgruppe, oder eine heterozyklische Gruppe, oder R₂ und R₃ bilden einen Teil einer zyklischen Struktur, die sie enthalten, wobei Substituenten, welche die zyklische Struktur bilden, unabhängig voneinander abgeleitet von einem Halogen, einer niederen Alkylgruppe, einer Hydroxylgruppe, einer Thiolgruppe, einer Amniogruppe, einer Alkoxygruppe, einer Arylgruppe, einer Arylthiolgruppe, einer Aryloxygruppe, einer Alkylthiolgruppe, einer Zykloalkylgruppe, oder einer heterozyklischen Gruppe in R₂ und R₃ sind.

2. Verfahren nach Anspruch 1, wobei der Stimulationsschritt das Aufbewahren der NK Zellen bei 38°C bis 40°C für 10 Stunden bis 30 Stunden umfasst, um auf diese eine Hochtemperatur- Stimulation anzuwenden.

3. Verfahren nach Anspruch 1 oder 2, wobei das Bisphosphonat-Derivat ausgewählt ist aus der Gruppe, bestehend aus Zoledronsäure, Pamidronsäure, Alendronsäure, Risedronsäure, Ibandronsäure, Incadronsäure, Etidronsäure, und eine Kombination davon.

4. Verfahren nach einem der Ansprüche 1 bis 3, wobei das Zytokin IL-2 ist.

5. Verfahren nach einem der Ansprüche 1 bis 4, wobei der anti-CD16 Antikörper auf einem Festphasenträger immobilisiert ist.

6. Verfahren nach einem der Ansprüche 1 bis 5, wobei die Kultivierungsperiode im Kulturschritt 9 Tage bis 21 Tage beträgt.

7. Verfahren nach einem der Ansprüche 1 bis 6, wobei das Blut peripheres Blut, Nabelschnurblut, oder Knochenmarksflüssigkeit ist.

8. Zusammensetzung für NK Zell Anreicherung umfassend einen anti-CD16 Antikörper, OK432, ein Bisphosphonat-Derivat oder Salz davon, oder ein Hydrat davon, und ein Zytokin,
wobei das Bisphosphonat-Derivat ein Stoff ist mit folgender Formel (1): 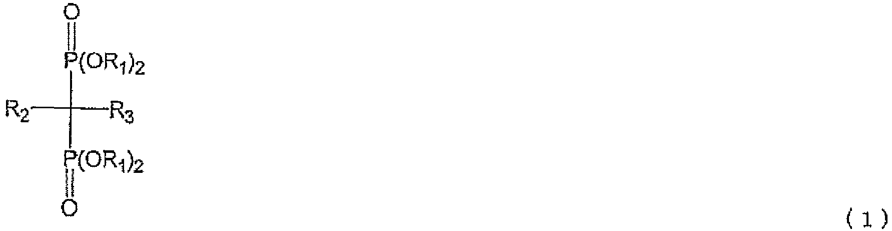 in der Formel, R₁ steht für ein Wasserstoffatom (H) oder eine niedere Alkyl Gruppe; R₂ und R₃ repräsentieren unabhängig voneinander ein Wasserstoffatom, Halogen, eine Hydroxylgruppe, eine Aminogruppe, eine Thiolgruppe, eine substituierte oder unsubstituierte Arylgruppe, eine substituierte oder unsubstituierte Alkylgruppe, eine niedere Alkylaminogruppe, eine Aralkylgruppe, eine Zykloalkylgruppe, oder eine heterozyklische Gruppe, oder R₂ und R₃ bilden einen Teil einer zyklischen Struktur, die sie enthalten, wobei Substituenten, welche die zyklische Struktur bilden, unabhängig voneinander abgeleitet von einem Halogen, einer niederen Alkylgruppe, einer Hydroxylgruppe, einer Thiolgruppe, einer Amniogruppe, einer Alkoxygruppe, einer Arylgruppe, einer Arylthiolgruppe, einer Aryloxygruppe, einer Alkylthiolgruppe, einer Zykloalkylgruppe, oder einer heterozyklischen Gruppe in R₂ und R₃ sind.

9. Zusammensetzung nach Anspruch 8, wobei das Bisphosphonat-Derivat ausgewählt ist aus der Gruppe, bestehend aus Zoledronsäure, Pamidronsäure, Alendronsäure, Risedronsäure, Ibandronsäure, Incadronsäure, Etidronsäure, und eine Kombination davon.

10. Zusammensetzung nach Anspruch 8 oder 9, wobei das Zytokin IL-2 ist.

11. Kit für die Produktion von NK-Zell angereichertem Blut umfassend einen anti-CD16 Antikörper, OK432, ein Bisphosphonat-Derivat oder Salz davon, oder ein Hydrat davon, und ein Zytokin,
wobei das Bisphosphonat-Derivat ein Stoff ist mit folgender Formel (1): 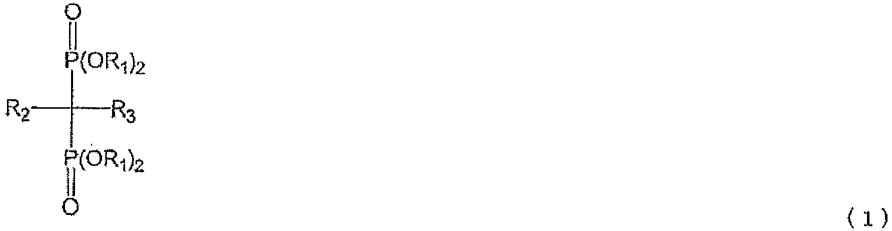 in der Formel, R₁ steht für ein Wasserstoffatom (H) oder eine niedere Alkyl Gruppe; R₂ und R₃ repräsentieren unabhängig voneinander ein Wasserstoffatom, Halogen, eine Hydroxylgruppe, eine Aminogruppe, eine Thiolgruppe, eine substituierte oder unsubstituierte Arylgruppe, eine substituierte oder unsubstituierte Alkylgruppe, eine niedere Alkylaminogruppe, eine Aralkylgruppe, eine Zykloalkylgruppe, oder eine heterozyklische Gruppe, oder R₂ und R₃ bilden einen Teil einer zyklischen Struktur, die sie enthalten, wobei Substituenten, welche die zyklische Struktur bilden, unabhängig voneinander abgeleitet von einem Halogen, einer niederen Alkylgruppe, einer Hydroxylgruppe, einer Thiolgruppe, einer Amniogruppe, einer Alkoxygruppe, einer Arylgruppe, einer Arylthiolgruppe, einer Aryloxygruppe, einer Alkylthiolgruppe, einer Zykloalkylgruppe, oder einer heterozyklischen Gruppe in R₂ und R₃ sind.

12. Kit nach Anspruch 11, wobei das Bisphosphonat-Derivat ausgewählt ist aus der Gruppe, bestehend aus Zoledronsäure, Pamidronsäure, Alendronsäure, Risedronsäure, Ibandronsäure, Incadronsäure, Etidronsäure, und eine Kombination davon.

13. Kit nach Anspruch 11 oder 12, wobei das Zytokin IL-2 ist.

## Revendications

1. Procédé de production d'un produit sanguin enrichi en cellules NK, comprenant : une étape de stimulation de cellules NK comprises dans du sang collecté à partir d'un organisme, avec des facteurs de stimulation de croissance de cellules NK comprenant au moins un anticorps anti-CD16, OK432 (Picibanil), un dérivé de biphosphonate ou un sel de celui-ci, ou un hydrate de celui-ci, et une cytokine ; et
une étape de culture de culture du sang à une température de cellule physiologique après l'étape de stimulation,
dans lequel le dérivé de biphosphonate est un composé représenté par la formule générale suivante 1 : 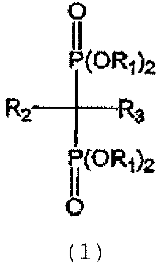 dans la formule, R₁ représente un atome d'hydrogène (H) ou un groupe alkyle inférieur ; R₂ et R₃ représentent chacun indépendamment un atome d'hydrogène, halogène, un groupe hydroxyle, un groupe amino, un groupe thiol, un groupe aryle substitué ou non substitué, un groupe alkyle substitué ou non substitué, un groupe alkylamino inférieur, un groupe aralkyle, un groupe cycloalkyle, ou un groupe hétérocyclique, ou R₂ et R₃ forment une partie d'une structure cyclique contenant ceux-ci dans laquelle les substituants où la structure cyclique sont chacun indépendamment dérivés d'un halogène, un groupe alkyle inférieur, un groupe hydroxyle, un groupe thiol, un groupe amino, un groupe alcoxy, un groupe aryle, un groupe arylthio, un groupe aryloxy, un groupe alkylthio, un groupe cycloalkyle, ou un groupe hétérocyclique dans R₂ et R₃.

2. Procédé selon la revendication 1, dans lequel l'étape de stimulation comprend le maintien des cellules NK à 38 °C à 40 °C pendant 10 heures à 30 heures pour appliquer à celles-ci une stimulation à haute température.

3. Procédé selon la revendication 1 ou 2, dans lequel le dérivé de biphosphonate est choisi dans le groupe constitué de l'acide zolédronique, l'acide pamidronique, l'acide alendronique, l'acide risédronique, l'acide ibandronique, l'acide incadronique, l'acide étidronique, et une combinaison de ceux-ci.

4. Procédé selon l'une quelconque des revendications 1 à 3, dans lequel la cytokine est IL-2.

5. Procédé selon l'une quelconque des revendications 1 à 4, dans lequel l'anticorps anti-CD16 est immobilisé sur un support en phase solide.

6. Procédé selon l'une quelconque des revendications 1 à 5, dans lequel la période de culture dans l'étape de culture est de 9 jours à 21 jours.

7. Procédé selon l'une quelconque des revendications 1 à 6, dans lequel le sang est du sang périphérique, du sang ombilical, ou du fluide de moelle osseuse.

8. Composition pour l'enrichissement en cellules NK comprenant un anticorps anti-CD16, OK432, un dérivé de biphosphonate ou un sel de celui-ci, ou un hydrate de celui-ci, et une cytokine,
où le dérivé de biphosphonate est un composé représenté par la formule générale 1 suivante : 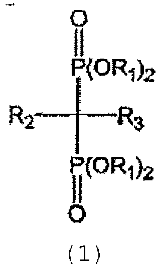 dans la formule, R₁ représente un atome d'hydrogène (H) ou un groupe alkyle inférieur ; R₂ et R₃ représentent chacun indépendamment un atome d'hydrogène, halogène, un groupe hydroxyle, un groupe amino, un groupe thiol, un groupe aryle substitué ou non substitué, un groupe alkyle substitué ou non substitué, un groupe alkylamino inférieur, un groupe aralkyle, un groupe cycloalkyle, ou un groupe hétérocyclique, ou R₂ et R₃ forment une partie d'une structure cyclique contenant ceux-ci dans laquelle les substituants formant la structure cyclique sont chacun indépendamment dérivés d'un halogène, un groupe alkyle inférieur, un groupe hydroxyle, un groupe thiol, un groupe amino, un groupe alcoxy, un groupe aryle, un groupe arylthio, un groupe aryloxy, un groupe alkylthio, un groupe cycloalkyle, ou un groupe hétérocyclique dans R₂ et R₃.

9. Composition selon la revendication 8, dans laquelle le dérivé de biphosphonate est choisi dans le groupe constitué de l'acide zolédronique, l'acide pamidronique, l'acide alendronique, l'acide risédronique, l'acide ibandronique, l'acide incadronique, l'acide étidronique, et une combinaison de ceux-ci.

10. Composition selon la revendication 8 ou 9, dans laquelle la cytokine est IL-2.

11. Kit pour la production de sang enrichi en cellules NK comprenant un anticorps anti-CD16, OK432, un dérivé de biphosphonate ou un sel de celui-ci, ou un hydrate de celui-ci, et une cytokine,
où le dérivé de biphosphonate est un composé représenté par la formule générale 1 suivante : 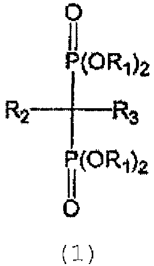 dans la formule, R₁ représente un atome d'hydrogène (H) ou un groupe alkyle inférieur ; R₂ et R₃ représentent chacun indépendamment un atome d'hydrogène, halogène, un groupe hydroxyle, un groupe amino, un groupe thiol, un groupe aryle substitué ou non substitué, un groupe alkyle substitué ou non substitué, un groupe alkylamino inférieur, un groupe aralkyle, un groupe cycloalkyle, ou un groupe hétérocyclique, ou R₂ et R₃ forment une partie d'une structure cyclique contenant ceux-ci dans laquelle les substituants formant la structure cyclique sont chacun indépendamment dérivés d'halogène, un groupe alkyle inférieur, un groupe hydroxyle, un groupe thiol, un groupe amino, un groupe alcoxy, un groupe aryle, un groupe arylthio, un groupe aryloxy, un groupe alkylthio, un groupe cycloalkyle, ou un groupe hétérocyclique dans R₂ et R₃.

12. Kit selon la revendication 11, dans lequel le dérivé de biphosphoziate est choisi dans le groupe constitué de l'acide zolédronique, l'acide pamidronique, l'acide alendronique, l'acide risédronique, l'acide ibandronique, l'acide incadronique, l'acide étidxonique, et une combinaison de ceux-ci.

13. Kit selon la revendication 11 ou 12, dans lequel la cytokine est IL-2.
